# EUROPÄISCHE PATENTSCHRIFT

(11) **EP 0 973 774 B1**
(45) Veröffentlichungstag und Bekanntmachung des Hinweises auf die Patenterteilung: **22.01.2003**
(21) Anmeldenummer: 98913595.9
(22) Anmeldetag: 26.02.1998
(51) Int. Cl.: C07D 487/04, A61K 31/505

(54) **1,5-DIHYDRO-PYRAZOLO 3,4-D]-PYRIMIDINON-DERIVATE**
1,5-DIHYDRO-PYRAZOLO 3,4-D]-PYRIMIDINONE DERIVATIVES
DERIVES DE 1,5-DIHYDRO-PYRAZOLO 3,4-D]-PYRIMIDINONE

(30) Priorität: 11.03.1997 DE 19709877
(43) Veröffentlichungstag der Anmeldung: 26.01.2000
(73) Patentinhaber: BAYER AG, 51368 Leverkusen (DE)
(72) Erfinder: HANING, Helmut, D-42113 Wuppertal (DE); NIEWÖHNER, Ulrich, D-42929 Wermelskirchen (DE); ROSENTRETER, Ulrich, D-42349 Wuppertal (DE); SCHENKE, Thomas, D-51469 Bergisch Gladbach (DE); KELDENICH, Jörg, D-42113 Wuppertal (DE); BISCHOFF, Erwin, D-42115 Wuppertal (DE); SCHLEMMER, Karl-Heinz, D-42113 Wuppertal (DE); SCHÜTZ, Helmuth, D-93049 Regensburg (DE); THOMAS, Günter, D-42115 Wuppertal (DE)
(86) Internationale Anmeldenummer: EP9801086
(87) Internationale Veröffentlichungsnummer: WO98040384

(56) Entgegenhaltungen:
- WO-A-96/28429
- DE-B- 1 153 023

## Beschreibung

Die vorliegende Erfindung betrifft 1,5-Dihydro-pyrazolo[3,4-d]-pyrimidinon-derivate, ein Verfahren zu ihrer Herstellung und ihre Verwendung in Arzneimitteln, insbesondere zur Behandlung von cardiovaskulären und cerebrovaskulären Erkrankungen, peripheren Gefäßerkrankungen und Krankheiten des Urogenitalsystems.

Phosphodiesterasen (PDE's) spielen eine wesentliche Rolle in der Regulation des intrazellulären cGMP und cAMP-Spiegels. Von den bisher beschriebenen Phosphodiesterase-Isoenzymgruppen PDE I bis PDE V [Nomenklatur nach Beavo and Reifsnyder (vgl. Beavo, J.A. and Reifsnyder, D.H.: Trends in Pharmacol. Sci 11, 150 - 155 (1990)] sind die Ca-Calmodulin aktivierte PDE I, die cGMP stimulierbare PDE II und die cGMP spezifische PDE V im wesentlichen für den Metabolismus von cGMP verantwortlich. Aufgrund der unterschiedlichen Verteilung dieser cGMP metabolisierenden PDE's im Gewebe sollten selektive Inhibitoren je nach Gewebsverteilung des entsprechenden Isoenzyms die c-GMP-Spiegel im entsprechenden Gewebe anheben. Dieses kann zu einer spezifischen antiaggregatorischen, antispastischen, gefäßdilatierenden, und/oder antiarrhythmischen Wirkung führen.

Außerdem sind aus US-5 294 612, US-4 211 731, US-3 211 732, WO 96/28448 und WO 96/28429 6-Heterocyclyl-pyrazolo[3,4-d]pyrimidin-3-one und 6-substituierte Pyrazolo[3,4-d]pyrimidin-4-one bekannt, die bei Bluthochdruck, Angina und Herzerkrankungen eingesetzt werden können.

Die vorliegende Erfindung betrifft jetzt 1,5-Dihydro-pyrazolo[3,4-b]-pyrimidon-derivate der allgemeinen Formel (I) 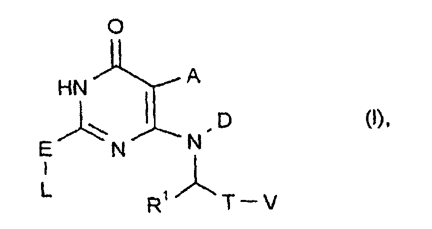 in welcher
- A und D: gemeinsam für einen Rest der Formel 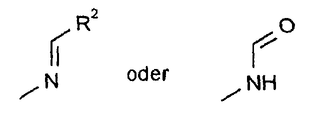 stehen
worin
R² Aryl bis 6 bis 10 Kohlenstoffatomen bedeutet, das gegebenenfalls bis zu 3-fach gleich oder verschieden durch Nitro, Cyano, Hxdroxyl, Trifluormethyl, Halogen, Carboxyl oder durch geradkettiges oder verzweigtes Acyl, Alkoxy oder Alkoxycarbonyl mit jeweils bis zu 6 Kohlenstoffatomen substituiert ist, oder
Wasserstoff, Trifluormethyl, Cyano, Carboxyl, oder geradkettiges oder verzweigtes Alkoxy oder Alkoxycarbonyl mit jeweils bis zu 8 Kohlenstoffatomen oder geradkettiges oder verzweigtes Alkyl mit bis zu 8 Kohlenstoffatomen bedeutet, das gegebenenfalls durch Hydroxy substituiert ist,
- R¹: für geradkettiges oder verzweigtes Acyl mit bis zu 4 Kohlenstoffatomen steht, oder
fiir geradkettiges oder verzweigtes Alkyl mit bis zu 10 Kohlenstoffatomen steht, das gegebenenfalls durch Hydroxy, Azido oder durch eine Gruppe der Formel-NR³R⁴ oder -OSO₂R⁵ substituiert ist,
worin
R³ und R⁴ gleich oder verschieden sind und Wasserstoff oder geradkettiges oder verzweigtes Alkyl mit bis zu 6 Kohlenstoffatomen bedeuten, oder
R³ und R⁴ gemeinsam mit dem Stickstoffatom einen 5 oder 6-gliedrigen gesättigten Heterocyclus bilden, der gegebenenfalls ein weiteres Heteroatom aus der Reihe S oder 0 oder einen Rest -NR⁶ enthalten kann,
worin
R⁶ Wasserstoff oder geradkettiges oder verzweigtes Alkyl ist bis zu 4 Kohlenstoffatomen bedeuten, und
R⁵ Phenyl oder geradkettiges oder verzweigtes Alkyl mit bis zu 5 Kohlenstoffatomen bedeutet,
- E: für eine geradkettige oder verzweigte Alkylen- oder Alkenylenkette mit jeweils bis zu 6 Kohlenstoffatomen steht, die gegebenenfalls durch Hydroxy substituiert sind, oder
für die C=O-Gruppe steht,
- L und V: gleich oder verschieden sind und für Aryl mit 6 bis 10 Kohlenstoffatomen oder fiir einen 5- bis 7-gliedrigen aromatischen, gegebenenfalls benzokondensierten Heterocyclus mit bis zu 3 Heteroatomen aus der Reihe S, N und/oder O stehen, die gegebenenfalls bis zu 3-fach gleich oder verschieden durch Halogen, Hydroxy, Nitro, Trifluormethyl, Carboxy, geradkettiges oder verzweigtes Alkyl, Alkoxy oder Alkoxycarbonyl mit jeweils bis zu 6 Kohlenstoffatomen oder durch eine Gruppe der Formel -(W)ₐ-NR⁷R⁸ substituiert sind,
worin
a eine Zahl 0 oder 1 bedeutet,
W einen Rest der Formel -CO oder -SO₂ bedeutet,
R⁷ und R⁸ gleich oder verschieden sind und die oben angegebene Bedeutung von R³ und R⁴ haben, und/oder die Cyclen gegebenenfalls durch Aryl mit 6 bis 10 Kohlenstoffatomen oder durch einen 5- bis 7-gliedrigen aromatischen, gegebenenfalls benzokondensierten Heterocyclus mit bis zu 3 Heteroatomen aus der Reihe S, N und/oder O substituiert sind, die ihrerseits gegebenenfalls bis zu 2-fach gleich oder verschieden durch Halogen, Hydroxy, Nitro, Carboxyl, Trifluormethyl oder durch geradkettiges oder verzweigtes Alkyl, Alkox oder Alkoxycarbonyl mit jeweils bis zu 5 Kohlenstoffatomen oder durch eine Gruppe der Formel -(W')_{b} -NR⁹R¹⁰ substituiert sind,
worin
b die oben angegebene Bedeutung von a hat und mit dieser gleich oder verschieden ist,
R⁹ und R¹⁰ die oben angegebene Bedeutung von R³ und R⁴ haben und mit dieser gleich oder verschieden sind,
W' die oben angegebene Bedeutung von W hat und mit dieser gleich oder verschieden ist, oder
- L: für einen Rest der Formel 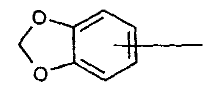 steht
- T: für einen Rest der Formel -CH₂X-Y- steht,
worin
X eine Bindung bedeutet oder ein Sauerstoff- oder Schwefelatom oder die -NH-Gruppe bedeutet,
Y eine geradkettige. oder verzweigte Alkylenkette mit bis zu 9 Kohlenstoffatomen bedeutet,
und deren Tautomere und Salze.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen der allgemeinen Formel (I) können in verschiedenen stereochemischen Formen auftreten, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Heterocyclus steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Pyridyl, Thienyl, Indolyl Furyl, Pyrrolyl, Thiazolyl, Oxazolyl, Imidazolyl, Morpholinyl oder Piperidyl. Bevorzugt sind Pyridyl, Thienyl, Indolyl und Furyl.

Bevorzugt sind Verbindungen der allgemeinen Formel (I),
in welcher
- A und D: gemeinsam für einen Rest der Formel 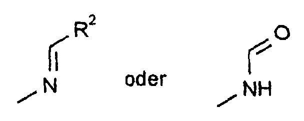 stehen
worin
R² Phenyl bedeutet, das gegebenenfalls bis zu 2-fach gleich oder verschieden durch Nitro, Cyano, Hxdroxyl, Trifluormethyl, Fluor, Chlor, Brom, Carboxyl oder durch geradkettiges oder verzweigtes Acyl, Alkoxy oder Alkoxycarbonyl mit jeweils bis zu 5 Kohlenstoffatomen substituiert ist, oder
Wasserstoff, Trifluormethyl, Cyano, Carboxyl, geradkettiges oder verzweigtes Alkoxy oder Alkoxycarbonyl mit jeweils bis zu 6 Kohlenstoffatomen oder geradkettiges oder verzweigtes Alkyl mit bis zu 6 Kohlenstoffatomen bedeutet, das gegebenenfalls durch Hydroxy substituiert ist,
- R¹: für geradkettiges oder verzweigtes Acyl mit bis zu 6 Kohlenstoffatomen steht, oder
für geradkettiges oder verzweigtes Alkyl mit bis zu 8 Kohlenstoffatomen steht, das gegebenenfalls durch Hydroxy, Azido oder durch eine Gruppe der Forrnel -NR³R⁴ oder O-SO₂-R⁵ substituiert ist,
worin
R³ und R⁴ gleich oder verschieden sind und
Wasserstoff oder geradkettiges oder verzweigtes Alkyl mit bis zu 5 Kohlenstoffatomen bedeuten, oder
R³ und R⁴ gemeinsam mit dem Stickstoffatom einen Morpholinyl-Piperidinyl- oder Piperazinylring bilden, wobei letzterer gegebenenfalls über die Stickstofffunktion durch geradkettiges oder verzweigtes Alkyl mit bis zu 3 Kohlenstoffatomen substituiert ist, und
R⁵ Phenyl oder geradkettiges oder verzweigtes Alkyl mit bis zu 4 Kohlenstoffatomen bedeutet,
- E: für eine geradkettige oder verzweigte Alkylen- oder Alkenylenkette mit jeweils bis zu 5 Kohlenstoffatomen steht, die gegebenenfalls durch Hydroxy substituiert sind, oder
für die C=O-Gruppe steht,
- L und V: gleich oder verschieden sind und für Phenyl, Naphthyl, Pyridyl, Thienyl, Indolyl oder Furyl stehen, die gegebenenfalls bis zu 3-fach gleich oder verschieden durch Fluor, Chlor, Brom, Trifluormethyl, Hydroxy, Nitro, Carboxy, geradkettiges oder verzweigtes Alkyl, Alkoxy oder Alkoxycarbonyl mit jeweils bis zu 5 Kohlenstoffatomen oder durch eine Gruppe der Formel -(W)ₐNR⁷R⁸ substituiert ist,
worin
a eine Zahl 0 oder 1 bedeutet,
W einen Rest der Formel -CO oder -SO₂ bedeutet,
R⁷ und R⁸ gleich oder verschieden sind und die oben angegebene Bedeutung von R³ und R⁴ haben, und/oder die Cyclen gegebenenfalls durch Naphthyl, Phenyl, Pyridyl, Indolyl, Thienyl oder Furyl gegebenenfalls durch Phenyl, Naphthyl, Pyridyl, Thienyl, Furyl, Pyrryl oder Pyrimidyl substituiert sind, die ihrerseits gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(W')_{b}NR⁹R¹⁰ substituiert sind,
worin
b die oben angegebene Bedeutung von a hat und mit dieser gleich oder verschieden ist,
W die oben angegebene Bedeutung von W hat und mit dieser gleich oder verschieden ist,
R⁹ und R¹⁰ die oben angegebene Bedeutung von R³ und R⁴ haben,
oder
- L: fiir einen Rest der Formel 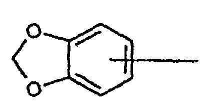 steht
- T: für einen Rest der Formel -CH₂-X-Y- steht,
worin
X eine Bindung bedeutet oder ein Sauerstoff- oder Schwefelatom oder die-NH-Gruppe bedeutet,
Y eine geradkettige oder verzweigte Alkylenkette mit bis zu 8 Kohlenstoffatomen bedeutet,
und deren Tautomere und Salze.

Besonders bevorzugt sind Verbindungen der allgemeinen Formel (I),
in welcher
- A and D: gemeinsam für einen Rest der Formel 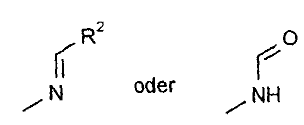 stehen
worin
R² Phenyl bedeutet, das gegebenenfalls bis zu 2-fach gleich oder verschieden durch Nitro, Cyano, Hxdroxyl, Trifluormethyl, Fluor, Chlor, Brom, Carboxyl oder durch geradkettiges oder verzweigtes Acyl, Akoxy oder Alkoxycarbonyl mit jeweils bis zu 4 Kohlenstoffatomen substituiert ist, oder
Wasserstoff, Trifluormethyl, Cyano, Carboxyl, geradkettiges oder verzweigtes Alkoxy oder Alkoxycarbonyl mit jeweils bis zu 5 Kohlenstoffatomen oder geradkettiges oder verzweigtes Alkyl mit bis zu 5 Kohlenstoffatomen bedeutet, das gegebenenfalls durch Hydroxy substituiert ist,
- R¹: für geradkettiges oder verzweigtes Acyl mit bis zu 5 Kohlenstoffatomen steht, oder
für geradkettiges oder verzweigtes Alkyl mit bis zu 6 Kohlenstoffatomen steht, das gegebenenfalls durch Hydroxy, Azido oder durch eine Gruppe der Formel-NR³R⁴ oder O-SO₂R⁵ substituiert ist,
worin
R³ und R⁴ gleich oder verschieden sind und Wasserstoff oder geradkettiges oder verzweigtes Alkyl mit bis zu 4 Kohlenstoffatomen bedeuten, oder
R³ und R⁴ gemeinsam mit dem Stickstoffatom einen Morpholinyl-, Piperdinyl- oder Piperazinylring bilden, wobei letzterer gegebenenfalls über die Stickstofffunktion durch Methyl substituiert ist, und
R⁵ Phenyl oder geradkettiges oder verzweigtes Alkyl mit bis zu 3 Kohlenstoffatomen bedeutet,
- E: für eine geradkettige oder verzweigte Alkylen- oder Alkenylenkette mit jeweils bis zu 5 Kohlenstoffatomen steht, die gegebenenfalls durch Hydroxy substituiert sind, oder
für die C=O-Gruppe steht,
- L und V: gleich oder verschieden sind und für Phenyl, Naphthyl, Furyl, Thienyl, Indolyl oder Pyridyl stehen, die gegebenenfalls bis zu 3-fach gleich oder verschieden durch Fluor, Chlor, Brom, Hydroxy, Nitro, Carboxy, geradkettiges oder verzweigtes Alkyl, Alkoxy oder Alkoxycarbonyl mit jeweils bis zu 4 Kohlenstoffatomen oder durch eine Gruppe der Formel -(W)ₐNR⁷R⁸ substituiert sind,
worin
a eine Zahl 0 oder 1 bedeutet,
W einen Rest der Formel -CO oder -SO₂ bedeutet,
R⁷ und R⁸ gleich oder verschieden sind und die oben angegebene Bedeutung von R³ und R⁴ haben, und/oder die Cyclen gegebenenfalls durch Phenyl, Pyridyl, Thienyl oder Furyl substituiert sind, die ihrerseits gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(W')_{b}NR⁹R¹⁰ substituiert sind,
worin
b die oben angegebene Bedeutung von a hat und mit dieser gleich oder verschieden ist,
W' die oben angegebene Bedeutung von W hat und mit dieser gleich oder verschieden ist,
R⁹ und R¹⁰ die oben angegebene Bedeutung von R³ und R⁴ haben,
oder
- L: für einen Rest der Formel 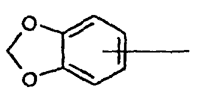 steht
- T: für einen Rest der Formel -CH₂-X-Y- steht,
worin
X eine Bindung bedeutet oder ein Sauerstoff- oder Schwefelatom oder die-NH-Gruppe bedeutet,
Y eine geradkettige oder verzweigte Alkylenkette mit bis zu 6 Kohlenstoffatomen bedeutet
und deren Tautomere und Salze.

Ganz besonders bevorzugt sind erfindungsgemäße Verbindungen der allgemeinen Formel (I),
in welcher
- A und D: gemeinsam für einen Rest der Formel 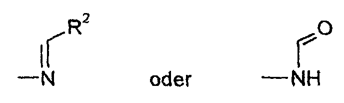 stehen,
worin
R² geradkettiges oder verzweigtes Alkyl oder Alkoxy mit jeweils bis zu 3 Kohlenstoffatomen bedeutet,
- R¹: für geradkettiges oder verzweigtes Alkyl mit bis zu 3 Kohlenstoffatomen steht oder
fiir einen Rest der Formel 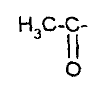 steht
- E: für eine geradkettige oder verzweigte Alkylenkette mit bis zu 3 Kohlenstoffatomen steht, die gegebenenfalls durch Hydroxyl substituiert ist,
- T: für einen Rest der Formel -CH₂-X-Y- steht,
worin
X eine Bindung bedeutet und
Y eine geradkettige oder verzweigte Alkylenkette mit bis zu 5 Kohlenstoffatomen bedeutet,
- V: für Phenyl steht,
- L: für einen Rest der Formel 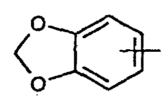 steht,
oder
für Phenyl steht, das gegebenenfalls bis zu 3-fach gleich oder verschieden durch Fluor, Chlor, Brom, Pyridyl, geradkettiges oder verzweigtes Alkyl oder Alkoxycarbonyl mit jeweils bis zu 3 Kohlenstoffatomen, Amino oder durch- einen Rest der Formel -SO₂-NHCH₃, 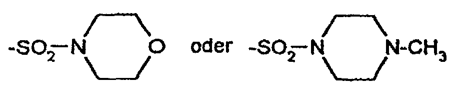 substituiert ist,
und/oder durch Phenyl oder durch Nitro substituiertes Phenyl substituiert sein kann,
und deren Tautomere und Salze.

Außerdem wurde ein Verfahren zur Herstellung der erfindungsgemäßen Verbindungen der allgemeinen Formel (I) gefunden, dadurch gekennzeichnet, daß man
[A] im Fall A und D gemeinsam für den Rest der Formel 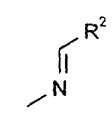 stehen,
   zunächst Verbindungen der allgemeinen Formel (II) 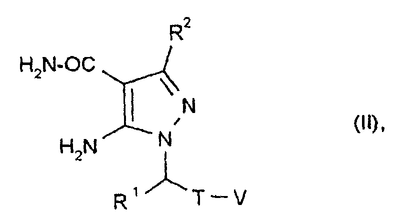 in welcher
   R¹, R², T und V die angegebene Bedeutung haben,
   durch Umsetzung mit Verbindungen der allgemeinen Formel (III)

   L-E-CO-Cl (III)

   in welcher
   E und L die angegebene Bedeutung haben,
   in inerten Lösemitteln und in Anwesenheit einer Base,
   in die Verbindungen der allgemeinen Formel (IV) 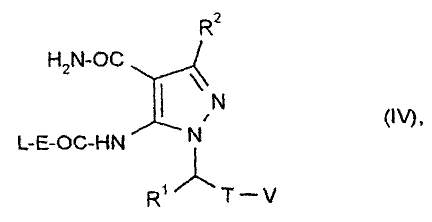 in welcher
   E, L, T, V, R¹ und R² die angegebene Bedeutung haben,
   überführt und anschließend mit Basen cyclisiert,
   oder
[B] Verbindungen der allgemeinen Formel (II) unter direkter Cyclisierung mit Verbindungen der allgemeinen Formel (IIIa)

   L-E-CO₂-R¹¹ (IIIa)

   in welcher
   E und L die oben angegebene Bedeutung haben,
   und
   - R¹¹: für Methyl oder Ethyl steht,
   umsetzt,
   und in einem zweiten Schritt in inerten Lösemitteln und in Anwesenheit einer Base cyclisiert,
   oder
[C] Verbindungen der allgemeinen Formel (V) 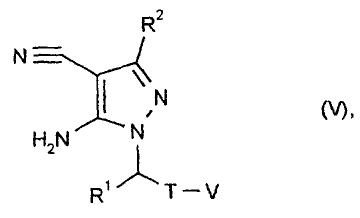 in welcher
   R¹, R², T und V die oben angegebene Bedeutung haben,
   zunächst durch Umsetzung mit Verbindungen der allgemeinen Formel (III) in inerten Lösemitteln und in Anwesenheit einer Base zu den Verbindungen der allgemeinen Formel (VI) 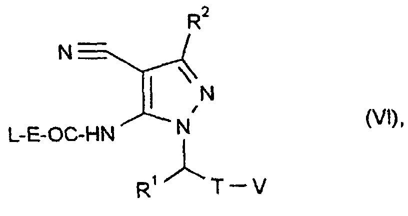 in welcher
   R¹, R², E, L, Tund V die oben angegebene Bedeutung haben,
   umsetzt,
   und in einem 2. Schritt in werten Lösemitteln und in Anwesenheit einer Base und eines Oxidationsmittels cyclisiert,
   oder
[D] im Fall, daß A und D gemeinsam für den Rest der Formel 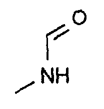 stehen,
   die entsprechenden Verbindungen der allgemeinen Formel (I), in welcher R² für Methoxy steht, in dem System Natriumjodid / Trimethylchlorsilan in inerten Lösemitteln umsetzt,
   und gegebenenfalls die unter R¹ aufgeführten Substituenten durch Folgereaktionen wie Acylierung, Oxidation, Substitution und/oder Reduktionen einführt bzw. derivatisiert,
   und ebenso die oben unter L und V aufgeführten Substituenten nach üblichen Methoden einführt und/oder variiert.

Das erfindungsgemäße Verfahren kann durch folgendes Formelschema beispielhaft erläutert werden:

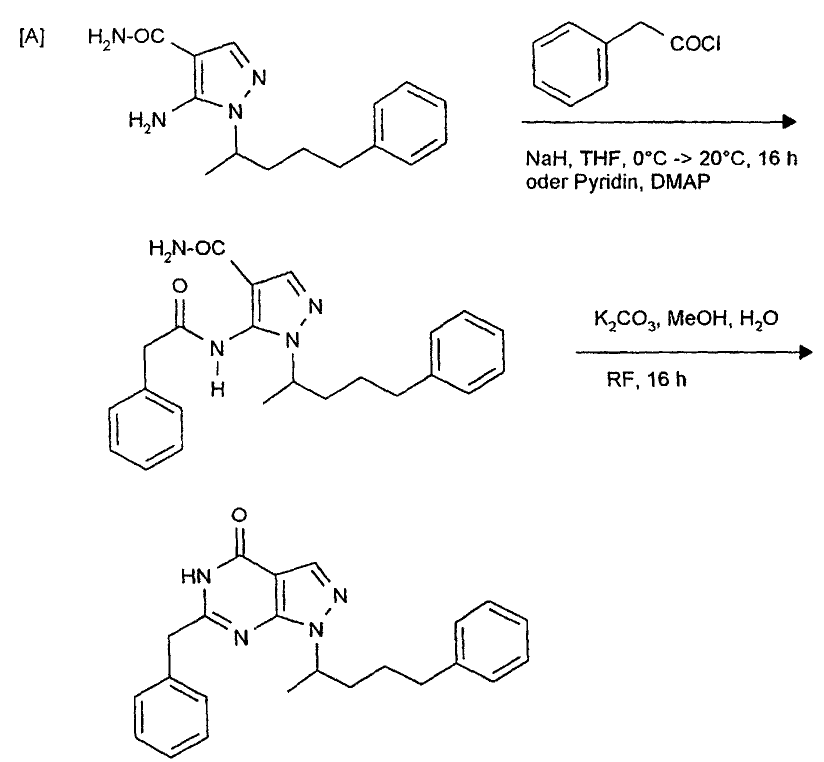

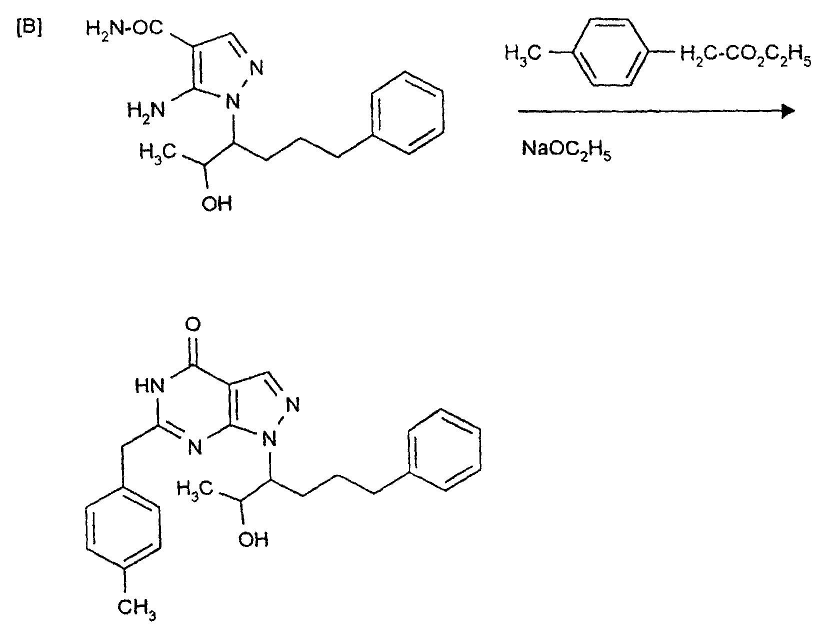

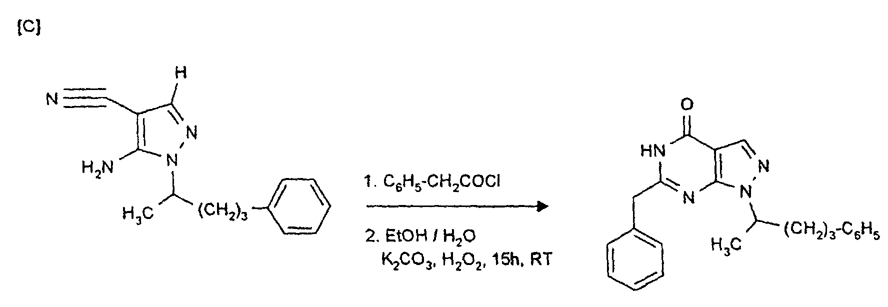

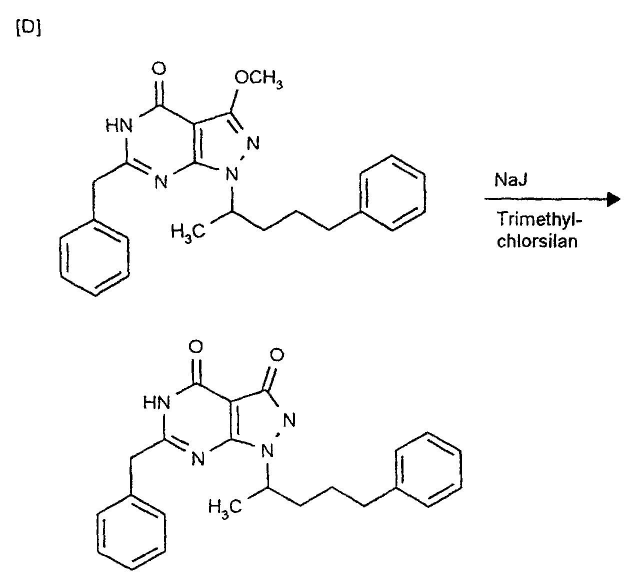

Für den ersten Schritt des Verfahrens [A] und für das Verfahren [C] eignen sich inerte organische Lösemittel, die sich unter den Reaktionsbedingungen nicht verändern Hierzu gehören bevorzugt Ether wie beispielsweise Diethylether, Dioxan, Tetrahydrofuran oder Glykoldimethylether oder Toluol, Hexamethylphosphorsäuretriamid und Pyridin Selbstverständlich ist es möglich, Gemische der Lösemittel einzusetzen. Besonders bevorzugt sind Tetrahydrofuran, Toluol oder Pyridin.

Als Basen eignen sich im allgemeine Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Natriumhydrid, Pyridin und/oder Dimethylaminopyridin.

Die Base wird im allgemeinen in einer Menge von 1 mol bis 4 mol, bevorzugt von 1,2 mol bis 3 mol jeweils bezogen auf 1 mol der Verbindungen der allgemeinen Formel (II) und (V) eingesetzt.

Die Reaktionstemperatur kann im allgemeinen in einem größeren Bereich variiert werden. Im allgemeinen arbeitet man in einem Bereich von -20°C bis 200°C, bevorzugt von 0°C bis 100°C.

In einer Variante wird die Umsetzung in Pyridin, dem eine katalytische Menge DMAP zugesetzt wird, durchgeführt. Gegebenenfalls kann noch Toluol zugefügt werden.

Als Lösemittel für die Cyclisierung eignen sich die üblichen organischen Lösemittel Hierzu gehören bevorzugt Alkohole wie Methanol, Ethanol, Propanol, Isopropanol, Butanol oder t-Butanol, oder Ether wie Tetrahydrofuran oder Dioxan, oder Dimethylformamid oder Dimethylsulfoxid. Besonders bevorzugt werden Alkohole wie Methanol, Ethanol, Propanol, Isopropanol oder t-Butanol verwendet. Ebenso ist es möglich, Gemische der genannten Lösemittel einzusetzen.

Als Basen eignen sich für die Cyclisierung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt sind Kaliumcarbonat, Natriumhydroxid und Kalium-tert.butanolat.

Bei der Durchführung der Cyclisierung wird die Base im allgemeinen in einer Menge von 2 bis 6 mol, bevorzugt von 3 bis 5 mol bezogen auf 1 mol der Verbindungen der Formel (IV), eingesetzt.

Als Oxidationsmittel für die Cyclisierung eignen sich beispielsweise Wasserstoffperoxid oder Natriumborat. Bevorzugt ist Wasserstoffperoxid.

Die Cyclisierung wird im allgemeinen in einem Temperaturbereich von 0°C bis 160°C, bevorzugt bei der Siedetemperatur des jeweiligen Lösemittels durchgeführt.

Die Cyclisierung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Lösemittel für das Verfahren [B] eignen sich die oben aufgeführten Alkohole, wobei Ethanol bevorzugt ist.

Als Basen für das Verfahren [B] eignen sich Alkoholate, wie beispielsweise Natriummethanolat, -ethanolat, isopropylat oder Kalium-tert.-butylat. Bevorzugt ist Natriumethanolat.

Die Base wird in einer Menge von 2 bis 8 mol, bevorzugt von 3 mol bis 6 mol jeweils bezogen auf 1 mol der Verbindungen der allgemeinen Formel (II) eingesetzt.

Als Lösemittel für das Verfahren [D] ist Acetonitril bevorzugt. Das Verfahren wird im allgemeinen unter Rückfluß und Normaldruck durchgeführt.

Die Umsetzung mit Alkylsulfonsäurechloriden erfolgt, ausgehend von den entsprechenden freien Hydroxyverbindungen, in einem der oben aufgeführten Lösemittel und einer der Basen, vorzugsweise mit Dichlormethan und Triethylamin in einem Temperaturbereich von -20C bis +20°C, vorzugsweise 0°C und Normaldruck.

Die Einführung des Azidrestes erfolgt im allgemeinen durch Umsetzung der entsprechenden Alkylsulfonyloxy substituierten Verbindungen mit Natriumazid in einem der oben aufgeführten Lösemittel, vorzugsweise Dimethylformamid, in einem Temperaturbereich von 50°C bis +120°C, vorzugsweise 100°C und Normaldruck.

Die Ketone werden ausgehend von den entsprechenden Hydroxyverbindungen nach bekannten Methoden (Swern-Oxidation oder Collins-Oxidation) hergestellt.

Die Variationen der Substituenten an den Aromaten werden nach bekannten Methoden durchgeführt.

Die enantiomerenreinen Verbindungen sind nach üblichen Methoden, beispielsweise durch Chromatographie der racemischen Verbindungen der allgemeinen Formel (I) auf chiralen Phasen oder durch die Verwendung chiraler Ausgangsverbindungen zugänglich.

Die Verbindungen der allgemeinen Formel (V) sind neu und können beispielsweise hergestellt werden, indem man im Fall R² ≠ OCH₃, Malonsäuredinitril mit Verbindungen der allgemeinen Formel (VII)

R²C(OC₂H₅)₃ (VII)

in welcher
R² die oben angegebene Bedeutung hat,
umsetzt und in einem zweiten Schritt mit Verbindungen der allgemeinen Formel (VIII) 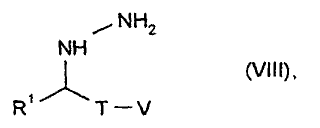 in welcher
R¹, T und V die angegebene Bedeutung haben,
in inerten Lösemitteln umsetzt,
und im Fall R² = OCH₃, die Verbindungen der allgemeinen Formel (VIII) direkt mit 1,1-Dimethoxy-2,2-dicyano-ethylen umsetzt.

Als Lösemittel für die einzelnen Schritte der Verfahren eignen sich die üblichen organischen Lösemittel, die sich unter den Reaktionsbedingungen nicht verändern. Hierzu gehören bevorzugt Alkohole wie Methanol, Ethanol, Propanol oder Isopropanol oder Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt sind im Fall R² ≠ OCH₃ Acetonitril und Pyridin im Fall von R² = OCH₃.

Das erfindungsgemäße Verfahren wird im allgemeinen in einem Temperaturbereich von 0°C bis +180°C, bevorzugt von +30°C bis +150°C durchgeführt.

Dies erfindungsgemäßen Verfahrenschritte werden im allgemeinen bei Normaldruck durchgeführt. Es ist aber auch möglich, bei Überdruck oder bei Unterdruck zu arbeiten (z.B. in einem Bereich von 0,5 bis 5 bar).

Die Verbindungen der allgemeinen Formeln (IV) und (VI) sind größtenteils neu und können wie oben beschrieben hergestellt werden.

Die Verbindungen der allgemeinen Formeln (III), (IIIa), (VII) und (VIII) sind an sich bekannt oder nach üblichen Methoden herstellbar.

Die Verbindungen der allgemeinen Formel (II) sind neu und können hergestellt werden, indem man die Verbindungen der allgemeinen Formel (V) mit einem der oben aufgeführten Oxidationsmittel, vorzugsweise Wasserstoffperoxid, in Anwesenheit von Ammoniak oder Dichlormethan oder mit einem Phasentransfer-Katalysator umsetzt.

Die Verbindungen der allgemeinen Formel (V) sind teilweise bekannt oder neu oder können nach üblichen Methoden hergestellt werden.

Die erfindungsgernäßen Verbindungen der allgemeinen Formel (I) zeigen ein nicht vorhersehbares, wertvolles pharmakologisches Wirkspektrum.

Sie inhibieren entweder eine oder mehrere der c-GMP metabolisierenden Phosphodiesterasen (PDE I, PDE II und PDE V). Dies führt zu einem differenzierten Anstieg von c-GMP. Eine Erhöhung des c-GMP-Spiegels, kann zu einer antithrombotischen, vasodilatorischen und/oder antiarrhythmischen Wirkung führen. Die differenzierende Wirkung wird von der Verteilung der Isoenzyme im Gewebe mitbestimmt.

Außerdem verstärken die erfindungsgemäßen Verbindungen die Wirkung von Substanzen, wie beispielsweise EDRF (Endothelium derived relaxing factor) und ANP (atrial natriuretic peptide), die den cGMP-Spiegel steigern.

Sie können daher in Arzneimitteln zur Behandlung von cardiovaskulären Erkrankungen wie beispielsweise zur Behandlung des Bluthochdrucks, neuronaler Hypertonie, stabiler und instabiler Angina, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, Angina pectoris, periphere Durchblutungsstörungen, Verhinderung von Restenosen nach Thrombolysetherapie, percutaner transluminaler Angioplastie (PTA), percutan transluminalen Koronarangioplastien (PTCA) und Bypass eingesetzt werden. Die relaxierende Wirkung auf glatter Muskulatur macht sie geeignet für die Behandlung von Erkrankungen des Urogenitalsystems wie Prostatahypertrophie, Impotenz und Inkontinenz. Weiterhin können sie auch Bedeutung für cerebrovaskuläre Erkrankungen haben.

### Aktivität der Phosphordiesterasen (PDE's)

Die c-GMP stimulierbare PDE II, die c-GMP hemmbare PDE III und die cAMP spezifische PDE IV wurden entweder aus Schweine- oder Rinderherzmyokard isoliert. Die Ca²⁺-Calmodulin stimulierbare PDE I wurde aus Schweineaorta, Schweinehirn oder bevorzugt aus Rinderaorto isoliert. Die c-GMP spezifische PDE V wurde aus Schweinedünndarm, Schweineaorta, humanen Blutplättchen und bevorzugt aus Rinderaorta gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990) und C. Lugman et al. Biochemical Pharmacology Vol. 35 1743-1751 (1986).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca 50% des Substrates umgesetzt werden. Um die cGMP stimulierbare PDE II zu testen, wird als Substrat ³HcAMP verwendet und dem Ansatz 10⁻⁶ mol/l nicht markiertes cGMP zugesetzt. Um die Ca-Calmodulinabhängige PDE I zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflußscintillationszähler quantitativ bestimmt. Es wird die Substanzkonzentration gemessen, bei der die Reaktionsgeschwindigkeit um 50% vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt. Für die Aktivitätsbestimmung der PDE2 wurde der [³H] cAMP SPA assay verwendet, wobei dem Reaktionsansatz 10⁻⁶ M cGMP zur Aktivierung des Enzyms zugegeben wurde. Für die Messung der PDE1 wurden Calmodulin 10⁻⁷ M und CaCl₂ 1µM zum Reaktionsansatz zugegeben. Die PDE5 wurde mit dem [³H] cGMP SPA assay gemessen.

Inhibition der Phosphodiesterasen in vitro

| Bsp.-Nr. | PDE I IC₅₀ [nM] | PDE II IC₅₀ [nM] | PDE V IC₅₀ [nM] |
|---|---|---|---|
| 9 | 300 | 300 | 300 |
| 10 | 300 | 500 | 50 |
| 11 | > 10.000 | 100 | 50 |
| 32 | 500 | 300 | 300 |
| 33 | 500 | 500 | 800 |

Die Verbindungen wurden auf antihypertensive Aktivität am narkotisierten Schwein untersucht.

Die erektionsauslösende Wirkung wurde am narkotisierten Kaninchen gemessen (C.G. Stief et al. World Journal Urology 1990, S. 233-236).

Die Substanzen wurden in Dosierungen 0,03 bis 10 mg/kg direkt in den Corpus cavernosum, intraduodenal, rektal, oral, transdermal oder intravenös appliziert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 10 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen:

### Beispiel I

### N'-(1-Methyl-4-phenyl-butyliden)-hydrazin-carbonsäure-tert-butyl ester

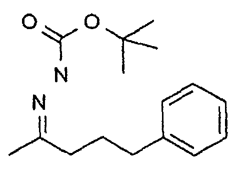

10.2 g (63 mmol) 5-Phenylpentan-2-on und 8.44 g (62 mmol) tert-Butylcarbazat werden in 100ml Heptan gelöst, 20min bei Raumtemperatur und 60 Minuten unter Rückfluß gerührt. Es wird 5 Stunden bei 4°C kristallisiert, abgesaugt, mit Pentan gewaschen und getrocknet.
Ausbeute: 14.6g (96%).
R_{f}=0.15 (PE/EE=9:1)

### Beispiel II

### (1-Methyl-4-phenyl-butyl)-hydrazin

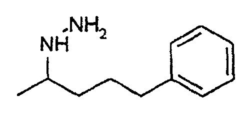

10.53 g (38 mmol) N'-(1-Methyl-4-phenyl-butyliden)-hydrazin-carbonsäure-tert-butyl ester werden in 30 ml THF und 40ml Methanol gelöst, mit 2.77 g (44 mmol) NaBH₃CN versetzt und 60 Minuten bei Raumtemperatur gerührt. Nach Zutropfen von 27.2 ml 6N HCl wird 60 Minuten refluxiert. Es wird mit 6N Natronlauge neutralisiert, die nichtwäßrigen Lösungsmittel werden abdestilliert und die wäßrige Phase wird dreimal mit Ethylacetat extrahiert. Die organische Phase wird über Na₂SO₄ getrocknet und das Lösungsmittel im Vakuum entfernt.
Ausbeute: 6.78 g (99%) gelbes Öl.
R_{f}=0.10 (PE/EE=5:1)

### Beispiel III

### 5-Amino-1-(1-Methyl-4-phenyl-butyl)-1H-pyrazol-4-carbonitril

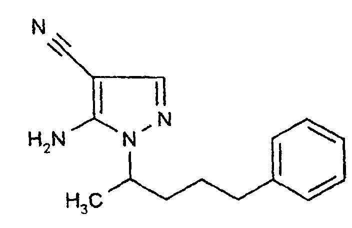

Zu 5.28 g (43 mmol) Ethoxymethylenmalonodinitril in 50 ml Methanol gibt man 7.70 g (43 mmol) (1-Methyl-4-phenyl-butyl)-hydrazin 10 ml Methanol und rührt 15 Minuten bei Raumtemperatur. Es wird 2 Stunden am Rückfluß erhitzt und dann das Lösungsmittel im Vakuum entfernt. Der Rückstand wird in 100 ml CH₂Cl₂ aufgenommen, die organische Phase wird mit ges. wäß. NaHCO₃-Lösung extrahiert, über Na₂SO₄ und einer Spatelspitze Aktivkohle getrocknet und das Lösungsmittel im Vakuum entfernt.
Ausbeute: 9.70 g (88%) rotes Öl.
R_{f}=0.15 (PE/EE=2:1)

### Beispiel IV

### 5-Amino-1-(1-Methyl-4-phenyl-butyl)-1H-pyrazol-4-carbonsäure-amid

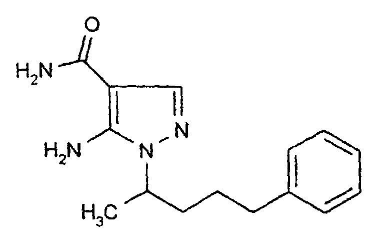

512 mg (2.02 mmol) 5-Amino-1-(1-Methyl-4-phenyl-butyl)-1*H*-pyrazole-4-carbonitril werden mit 25 ml konz. wäß. NH₃, 20 ml Ethanol und 5 ml 30% H₂O₂ 3 Stunden bei Raumtemperatur gerührt. Das nichtwäßrige Lösungsmittel wird im Vakuum entfernt, der ausgefallene Feststoff abgesaugt und getrocknet.
Ausbeute: 408 mg (75%) weißer Feststoff.
Smp.: 128°C

### Beispiel V

### 2-(1-Ethoxy-propylidene)-malonodinitril

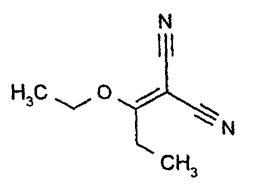

16.41 g (249 mmol) Malonodinitril und 43.8 g (249 mmol) Triethylorthopropionat werden 4.5 Stunden am Rückfluß erhitzt. Die Reaktionsmischung wird auf Raumtemperatur gekühlt und im Vakuum destilliert
Sdp.: 90°C (3 mbar)
Ausbeute: 28.4g (75%)

### Beispiel VI

### 2-(1-Ethoxy-ethylidene)-malonodinitril

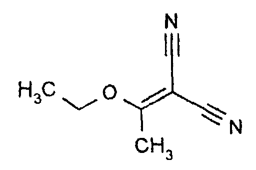

8.26 g (125 mmol) Malonodinitril und 20.25 g (125 mmol) Triethylorthoacetat werden 5 Stunden am Rückfluß erhitzt. Nach dem Abkühlen wird im Vakuum getrocknet.
Ausbeute: 16.0g (quantitativ)

### Beispiel VII

### 5-Amino-3-ethyl-1-(1-methyl-4-phenyl-butyl)-1-H-pyrazol-4-carbonitril

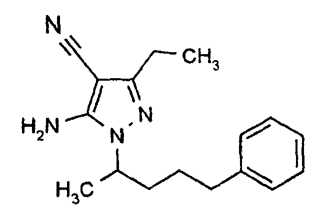

5 g (28 mmol) (1-Methyl-4-phenyl-butyl)-hydrazin und 4.17 g (28mmol) 2-(1-Ethoxypropyliden)-malonodinitril werden in 150 ml Methanol 2.25 Stunden am Rückfluß erhitzt. Das Lösungsmittel wird im Vakuum entfernt, der Rückstand in 100 ml CH₂Cl₂ aufgenommen, mit 0.1N HCl und gesättigter wäßriger NaHCO₃ Lösung extrahiert, über Na₂SO₄ getrocknet und das Lösungsmittel im Vakuum entfernt.
Ausbeute. 7.2 g (99%) rotes Öl.
R_{f}= 0.15 (PE/EE=2:1).

### Beispiel VIII

### 2-Hydroxy-6-phenyl-hexan-3-on

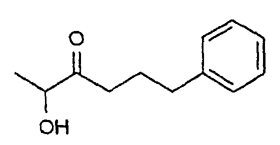

7.3 ml (55 mmol) Trimethylsilylcyanid werden unter Argon auf 0°C gekühlt und nacheinander mit 2 Tropfen BF₃.OEt₂ und 3.1 ml (55 mmol) Acetaldehyd versetzt. Man rührt 1.5h bei Raumtemperatur und löst das entstandene Cyanhydrin in 10 ml Ether. 1.3 g (55 mmol) Magnesium werden in 4 ml Ether unter Argon mit 1 ml (6.6 mmol) 3-Phenyl-1-propylbromid versetzt. Nach Abklingen der spontanen Reaktion werden weitere 7.4 ml (48.7 mmol) 3-Phenyl-1-propylbromid gelöst in 10 ml Ether so schnell zugetropft, daß ein gelinder Rückfluß erhalten bleibt. Nach beendeter Zugabe wird 30 Minuten am Rückfluß erhitzt und dann auf 0°C abgekühlt. Die Lösung des Cyanhydrins wird bei 0°C zugetropft und die Reaktionsmischung bei Raumtemperatur 15 Stunden gerührt Die Reaktionsmischung wird mit 100 ml Ether verdünnt, auf eine Mischung aus 300 g Eis und 15 ml konz H₂SO₄ gegossen und 3 Stunden bei Raumtemperatur gerührt.. Die etherische Phase wird dreimal mit 10%HCI und einmal mit gesättigter Kochsalzlösung extrahiert, über Na₂SO₄ getrocknet und das Lösungsmittel im Vakuum entfernt.
Ausbeute: 7.15 g (68%) farbloses Öl
R_{f}: 0.12 (PE/EE=9:1)

### Beispiel IX

### N'-(1-Methyl-4-phenyl-butylidene)-hydrazin-carbonsäure-tert-butylester

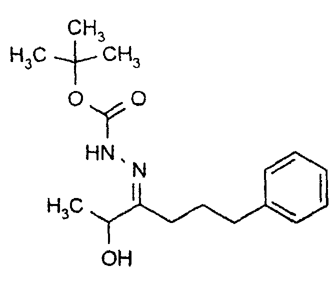

8.1 g (42 mmol) 2-Hydroxy-6-phenylhexan-3-on und 5.68 g tert-Butylcarbazat (43 mmol) werden in 70 ml Heptan und 35 ml THF gelöst, 30 Minuten bei Raumtemperatur und 1.5 Stunden am Rückfluß gerührt. Das Lösungsmittel wird im Vakuum entfernt.
Ausbeute; 12.81 g (99%) gelbes Öl.
R_{f}:0.10 (PE/EE=9:1).

### Beispiel X

### 5-Amino-1-[1-(1-hydroxy-ethyl)4-phenyl-butyl]-1H-pyrazol-4-carbonitril

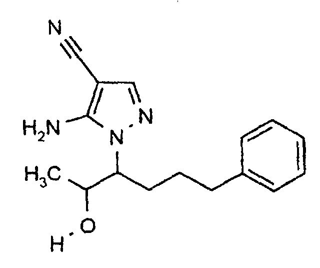

12.79 g (42 mmol) N'-(1-Methyl-4-phenyl-butylidene)-hydrazincarbonsäure-tert-butylester werden in 30 ml THF und 40 ml Methanol gelöst, mit 6.08 g (97 mmol) NaBH₃CN versetzt und 60 Minuten bei Raumtemperatur gerührt. Nach Zutropfen von 35 ml 6N HCl wird 60 Minuten refluxiert. Es wird mit 6N Natronlauge neutralisiert und dreimal mit CH₂Cl₂ extrahiert. Die organische Phase wird über Na₂SO₄ getrocknet und das Lösungsmittel im Vakuum entfernt. Man erhält 7.38 g eines schwach gelben Öls. 6.5 g dieses Öls werden mit 3.93 g (32 mmol) Ethoxymethylenemalonodinitril in 200 ml Methanol zunächst eine Stunde bei Raumtemperatur gerührt und dann 2 Stunden refluxiert. Nach dem Abkühlen wird das Lösungsmittel im Vakuum entfernt. Nach chromatographischer Reinigung (PE/EE=2:1) erhält man 5.28g eines farblosen Öls.
R_{f}: 0.10 (PE/EE=2:1).

### Beispiel XI

### 5-Amino-1-[1-(1-hydroxy-ethyl)-4-phenyl-butyl]-1H-pyrazol-4-carbonsäureamid

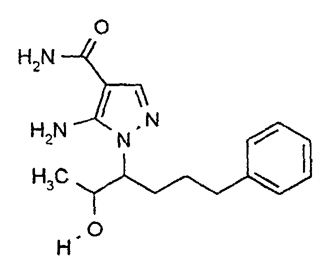

3.9 g (13.7 mmol) S-Amino-1-[1-(1-hydroxy-ethyl)-4-phenyl-butyl]-1H-pyrazol-4-carbonitril und 960 mg Tetrabutylammoniumhydrogensulfat werden in 28 ml CH₂Cl₂ gelöst, mit 5.5 ml 5M NaOH und 7ml 30% H₂O₂ versetzt und 4 Stunden bei Raumtemperatur gerührt. Die Phasen werden getrennt, die organische Phase zweimal mit Wasser extrahiert, die organische Phase über Na₂SO₄ getrocknet und das Lösungsmittel im Vakuum entfernt.
Ausbeute: 3.8g (92%)
Smp.:116°C(PE/EE)

### Beispiel XII

### 5-Amino-1-[1-(1-hydroxy-ethyl)-4-phenyl-butyl]-3-methyl-1H-pyrazol-4-carbonitril

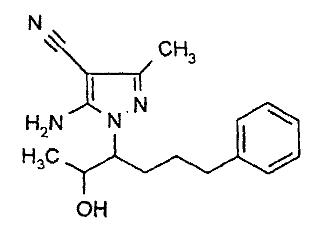

12.79 g (42 mmol) N'-(1-Methyl-4-phenyl-butylidene)-hydrazincarbonsäure-test-butylester werden in 30 ml THF und 40 ml Methanol gelöst, mit 6.08 g (97 mmol) NaBH₃CN versetzt und 60 Minuten bei Raumtemperatur gerührt. Nach Zutropfen von 35 ml 6N HCl wird 60 Minuten refluxiert. Es wird mit 6N Natronlauge neutralisiert und dreimal mit CH₂Cl₂ extrahiert Die organische Phase wird über Na₂SO₄ getrocknet und das Lösungsmittel im Vakuum entfernt. Man erhält 7.38 g eines schwach gelben Öls. 2,64 g dieses Öls (11.3 mmol) und 1.4g 2-(1-Ethoxy-ethyliden)-malondinitril (11.3 mmol) werden in 20 ml Methanol 2 Stunden am Rückfluß erhitzt. Das Lösungsmittel wird im Vakuum entfernt.
3.73g rotes Öls.
R_{f}: 0.11 (PE/EE=2;1)

### Beispiel XIII

### 5-Amino-1-[1-(1-hydroxy-ethyl)-4-phenyl-butyl]-3-methyl-1H-pyrazol-4-carbonsäureamid

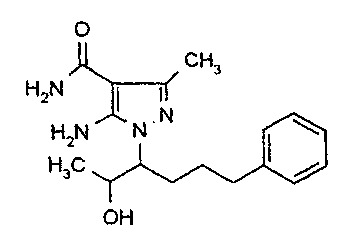

3.7 g (12.37 mmol) 5-Amino-1-[1-(1-hydroxy-ethyl)-4-phenyl-butyl]-3methyl-1H-pyrazol-4-carbonitril und 950 mg Tetrabutylammoniumhydrogensulfat werden in 25 ml CH₂Cl₂ gelöst, mit 5.5 ml 5M NaOH und 7ml 30% H₂O₂ versetzt und 15 Stunden bei Raumtemperatur gerührt. Die Phasen werden getrennt, die organische Phase zweimal mit Wasser extrahiert, die organische Phase über Na₂SO₄ getrocknet und das Lösungsmittel im Vakuum entfernt. Ausbeute: 3.24g (83%), R_{f}= 0,1 (Petrolether/Essigester =1:1)

### Beispiel XIV

### 5-Amino-4-cyano-3-methoxy-1-[(5-phenyl)-pent-2-yl]-pyrazol

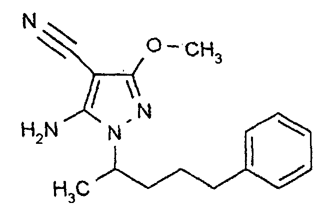

3,6 g 2-Hydrazino-5-phenylpentan werden in 40 ml Methanol gelöst und mit 2,8 ml Triethylamin versetzt. Dann werden 2,8 g 1,1-Dimethoxy-2,2-dicyanoethylen bei ca 0°C zugegeben. Die Reaktionslösung wird auf ca 20°C erwärmt und 1,5 h bei ca 20°C gerührt Zur Aufarbeitung wird mit Wasser verdünnt, mit Zitronensäure angesäuert und mit Dichlormethan zweimal extrahiert. Die vereinigten Dichlormethanphasen werden mit Magnesiumsulfat getrocknet und i.V. eingedampft. Man erhält 5,1 g gelbes Öl, das langsam durchkristallisiert. Das Öl wird an Kieselgel (Merck Si 60 0,04-0,063 mm) mit einem Petrolether/Ethylacetat-Gemisch im Verhältnis von 5:1 bis 1:1 chromatographiert. Man erhält so eine Fraktion, die nach Eindampfen i.V. 4,6 g (= 80,9% d.Th.) 5-Amino-4-cyano-3-methoxy-1-[(5-phenyl)-pent-2-yl]-pyrazol ergibt.
NMR(400MHZ, CD3OD): 1,3[3]d J=BHz; 1,4-1,65[3]m; 1,85-1,95[1]m; 2,5-2,65[2]m; 3,85[3]s; 4,1-4,2[1]m; 7,1-7,15[3]m; 7,2-7,25[2]m
DC RF-Wert= 0,6 ; Laufmittel: Petroether/Ethylacetat 1:1; Merck Si60

### Beispiel XV

### 5-Amino-4-cyano-3-methoxy-1-[2-hydroxy-6-phenyl-hex-3-yl]-pyrazol

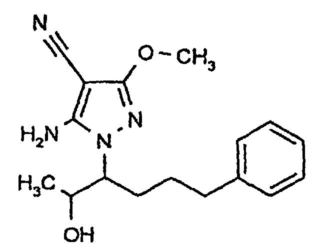

4,2 g 3-Hydrazino-2-hydroxy-6-phenylhexan werden werden in 20 ml Methanol gelöst und mit 1,38 ml Triethylamin versetzt Dann werden 1,38 g 1,1-Dimethoxy-2,2-dicyanoethylen zugegeben. Die Reaktionslösung wird 2 h bei ca 20°C gerührt. Zur Aufarbeitung wird mit Zitronensäurelösung und Ethylacetat verdünnt und mit Ethylacetat dreimal extrahiert. Die vereinigten Ethylacetatphasen werden mit Magnesiumsulfat getrocknet und i.V. eingedampft. Der Eindampfrückstand wird an Kieselgel (Merck Si 60 0,04-0,063 mm) mit einem Cyclohexan/Ethylacetat-Gemisch im Verhältnis von 4:1 bis 1:1 chromatographiert. Man erhält so eine Fraktion, die nach Eindampfen i.V. 0,959 g (= 30,5 % d.Th.) 5-Amino-4-cyano-3-methoxy-1-[2-hydroxy-6-phenyl-hex-3-yl]-pyrazol als Dia-stereomerengemisch ergibt.
DC RF-Wert= 0,2 ; Laufmittel: Dichlormethan/Methanol 10:1; Merck Si60 Art.Nr. 1.05719

### Beispiel XXX

### 5-Amino-3-ethyl-1-(1-methyl-4-phenyl-butyl)-1-H-pyrazol-4-carbonsäureamid

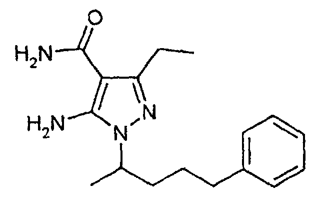

5,08 g (18 mmol) 5-Amino-3-ethyl-1-(1-methyl-4-phenyl-butyl)-1-*H*-pyrazol-4-carbonitril werden in 100 ml Ethanol gelöst und nach Zugabe von 100 ml konz. NH₃-Lösung (25 %) und 50 ml Wasserstoffperoxidlösung (30 %) bei Raumtemperatur 16 Stunden gerührt. Nach Entfernen der nichtwäßrigen Lösungsmittel wird mit 1N HCl auf pH 5 eingestellt. Die wäßrige Phase wird dreimal mit Dichlormethan extrahiert, die organische Phase über Natriumsulfat getrocknet und einrotiert. Man erhält 3,58 g braunes Öl (66 %).

### Beispiel XXXI

### 5-Amino-1-[1-(1-hydroxy-ethyl)-4-phenyl-butyl]-3-ethyl-1H-pyrazol-4-carbonsäureamid

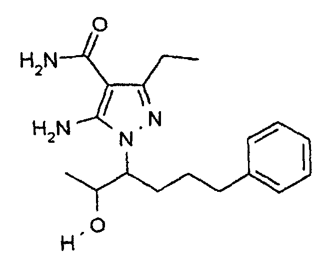

12,79 g (42 mmol) N'-(1-Methyl-4-phenyl-butyliden)-hydrazincarbonsäure*-tert*.butyl-ester werden in 30 ml THF und 40 ml Methanol gelöst, mit 6,08 g (97 mmol) NaBH₃CN versetzt und 60 Minuten bei Raumtemperatur gerührt. Nach Zutropfen von 35 ml 6N HCI wird 60 Minuten refluxiert. Es wird mit 6N Natronlauge neutralisiert und dreimal mit CH₂Cl₂ extrahiert. Die organische Phase wird über Na₂SO₄ getrocknet und das Lösungsmittel im Vakuum entfernt. Man erhält 7,38 g eines schwach gelben Öls. 3,00 g dieses Öls (14,4 mmol) und 2,50 g 2-(1-Ethoxy-propylidene)-malonodinitril (16,6 mmol) werden in 25 ml Methanol 3 Stunden am Rückfluß erhitzt Das Lösungsmittel wird im Vakuum entfernt. 4,238 g rotes Öl. R_{f}= 0.15. (PE/EE = 2:1). Dieses Öl wird in 136 ml Ethanol gelöst und nach Zugabe von 170 ml konz. NH₃-Lösung und 34 ml 30 % Wasserstoffperoxidlösung bei Raumtemperatur 18 Stunden gerührt. Nach Entfernen der nichtwäßrigen Lösungsmittel im Vakuum wird dreimal mit Dichlormethan extrahiert, die organische Phase über Natriumsulfat getrocknet und das Lösungsmittel im Vakuum entfernt. Man erhält 2,55 g (53 %) rotes Öl. R_{f}= 0.15 (PE/EE = 1:1).

### Herstellungsbeispiele

### Beispiel 1

### 6-Benzyl-1-(1-methyl-4-phenyl-butyl)-1,5-dihydro-pyrazolo[3,4-d]pyrimidin-4-one

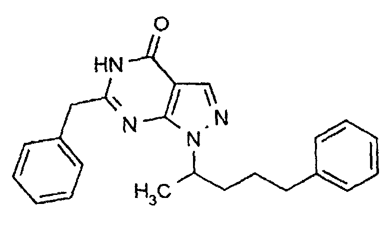

870 mg (3,42 mmol) 5-Amino-1-(1-Methyl-4-phenyl-butyl)-1H-pyrazol-4-carbonitril und 70 mg DMAP werden in 5 ml Pyridin gelöst und mit einer Lösung von 582 mg (3,78 mmol) Phenylessigsäurechlorid in 2 ml Toluol tropfenweise versetzt. Die Lösung wird 30 Minuten bei Raumtemperatur und 2 Stunden bei 60°C gerührt, das Lösungsmittel im Vakuum entfernt, der Rückstand in 50 ml CH₂Cl₂ aufgenommen, die organische Phase mit 1N HCl und ges. wäß. NaHCO₃ extrahiert, über Na₂SO₄ getrocknet und das Lösungsmittel im Vakuum entfernt.

Der Rückstand wird in 30 ml Wasser und 16 ml Ethanol mit 2 g K₂CO₃ und 4 ml 30 % H₂O₂ 15 h am Rückfluß erhitzt. Die Reaktionsmischung wird nach Acidifizieren mit 1N HCl mit Ethylacetat extrahiert, die organische Phase über Na₂SO₄ getrocknet und das Lösungsmittel im Vakuum entfernt. Chromatographische Reinigung (Petrolether/Essigester) ergibt 450 mg (35 %) Feststoff. Smp.: 136°C.

In Analogie zur Vorschrift des Beispiels 1 erhält man die in der Tabelle 1 aufgeführten Verbindungen:

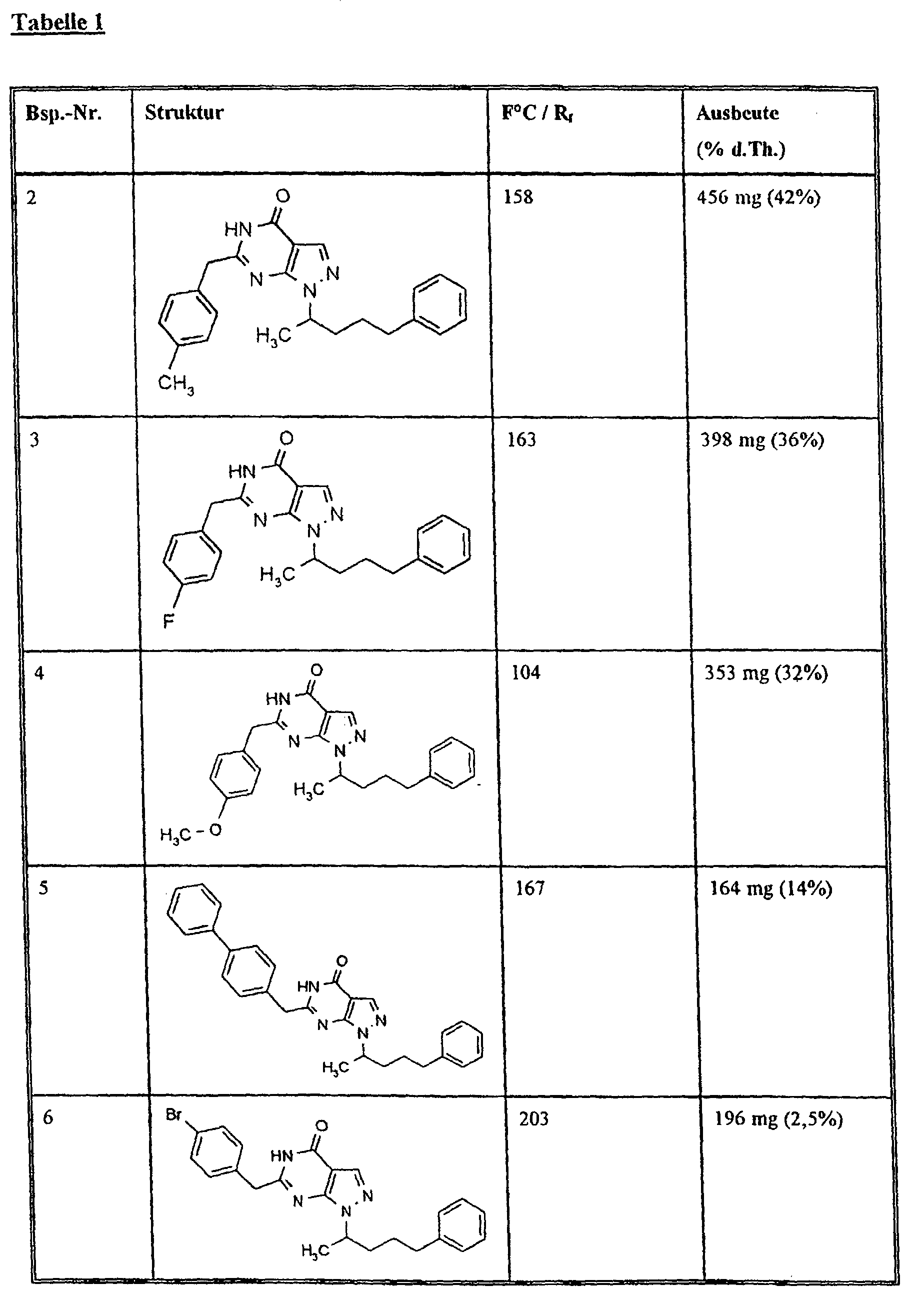

### Beispiel 7

### 6-(4-Chlor-benzyl)-1-(1-methyl-4-phenyl-butyl)-1,5-dihydro-pyrazolo[3,4-d]pyrimidin-4-on

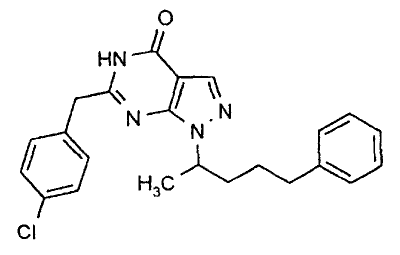

400 mg (1,47 mmol) 5-Amino-1-(1-Methyl-4-phenyl-butyl)-1*H*-pyrazol-4-carbonsäureamid und 80 mg DMAP werden in 12 ml Pyridin gelöst und mit einer Lösung von 457 mg (2,43 mmol) p-Chlorphenylessigsäurechlorid in 1 ml Toluol tropfenweise versetzt. Die Lösung wird 3,5 Stunden bei 60°C gerührt, das Lösungsmittel im Vakuum entfernt, der Rückstand in 50 ml CH₂Cl₂ aufgenommen, die organische Phase mit 1N HCl und ges. wäß. NaHCO₃ extrahiert, über Na₂SO₄ getrocknet und das Lösungsmittel im Vakuum entfernt.

Der Rückstand wird in 4 ml Wasser, 15 ml Methanol und 7 ml Ethanol mit 700 mg NaOH 3 h am Rückfluß erhitzt. Die nichtwäßrigen Lösungsmittel werden im Vakuum entfernt, der Rückstand nach Acidifizieren mit 1N HCl mit Ethylacetat extrahiert, die organische Phase über Na₂SO₄ getrocknet und das Lösungsmittel im Vakuum entfernt. Chromatographische Reinigung (Petrolether/Essigester) ergibt 380 mg (64 %).
Smp.: 168°C.

In Analogie zur Vorschrift des Beispiels 7 erhält man die in der Tabelle 2 aufgeführten Verbindungen:

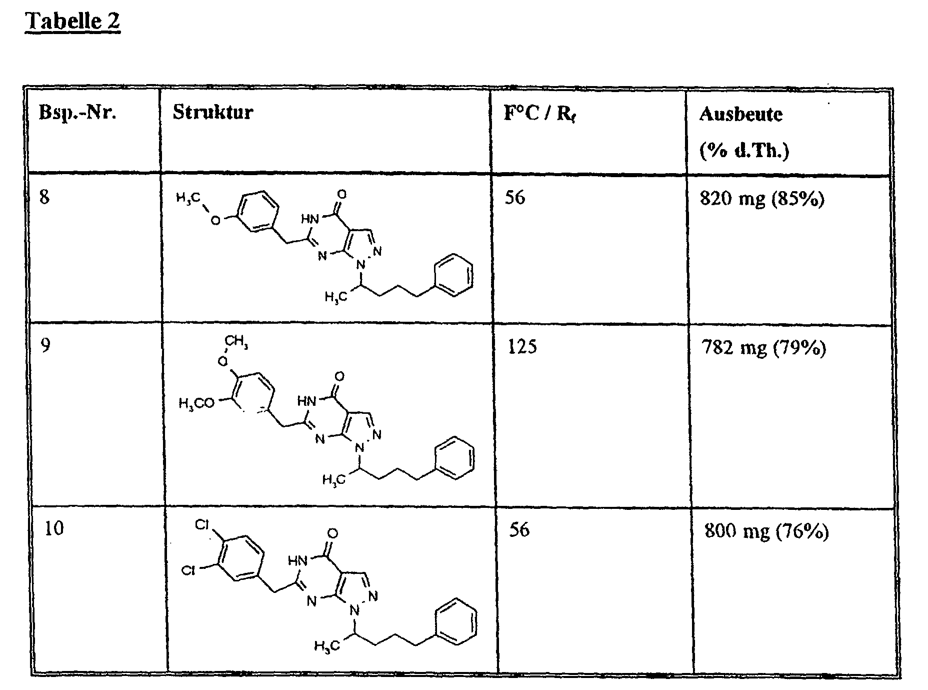

### Beispiel 11

### 6-(4-Brom-benzyl)-1-(1-methyl-4-phenyl-butyl)-3-ethyl-1,5-dihydro-pyrazol[3,4-d]pyrimidin-4-on

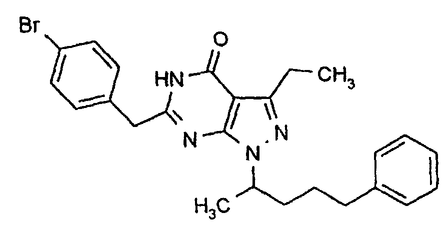

2,93 g (10,39 mmol) 5-Amino-3-ethyl-1-(1-methyl-4-phenyl-butyl)-1-*H*-pyrazol-4-carbonitril und 250 mg 4-Dimethylaminopyridin werden in 24 ml Pyridin gelöst und mit einer Lösung von 2,91 g (12,54 mmol) 4-Bromphenylacetylchlorid in 7 ml Toluol versetzt. Man rührt 2,5 Stunden bei 60°C, entfernt das Lösungsmittel im Vakuum und nimmt in 100 ml CH₂Cl₂ auf. Man extrahiert mit 1N HCI, gesättigter NaHCO₃-Lösung und Wasser, trocknet über Na₂SO₄ und entfernt das Lösungsmittel im Vakuum. Das Rohprodukt wird in 24 ml Pyridin gelöst und mit einer Lösung von 2 g (8,62 mmol) 4-Bromphenylacetylchlorid in 5 ml Toluol versetzt. Man rührt 2,5 Stunden bei 60°C, entfernt das Lösungsmittel im Vakuum und nimmt in 100 ml CH₂Cl₂ auf. Man extrahiert mit 1N HCl, gesättigter NaHCO₃-Lösung und Wasser, trocknet über Na₂SO₄ und entfernt das Lösungsmittel im Vakuum.

Das Reaktionsprodukt wird in 370 ml Ethanol gelöst und nach Zugabe von 600 ml 1N NaOH und 70 ml H₂O₂ (30 %) 5 Stunden bei 90°C gerührt. Das Lösungsmittel wird im Vakuum entfernt, nach Zugabe von 1N HCl dreimal mit CH₂Cl₂ extrahiert, über Na₂SO₄ getrocknet und das Lösungsmittel im Vakuum entfernt. Chromatographische Reinigung (CH₂Cl₂/Methanol = 20:1) und Kristallisation aus Ether ergeben 150 mg (3 %) farblosen Feststoff.
Smp.: 112°C.

### Beispiel 12

### 6-(3'-Amino-biphenyl-4-yl-methyl)-3-ethyl-1-(1-methyl-4-phenyl-butyl)-1,5-dihydropyrazolo[3,4-d]pyrimidin-4-on

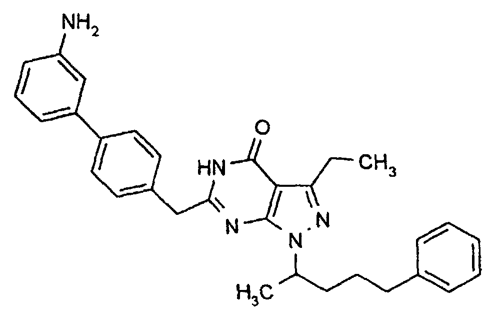

Zu einer Lösung von 1,66 g (3,47 mmol) 6-(4-Bromo-benzyl)-1-(1-methyl-4-phenyl-butyl)-3-ethyl-1,5-dihydro-pyrazolo[3,4-*d*]pyrimidin-4-on in 38 ml THF gibt man unter Argon 697 mg (4,5 mmol) 3-Aminophenylboronsäuremonohydrat und 150 mg Tetrakistriphenylphosphin-palladium und rührt eine Stunde bei 70°C. Nach Zugabe von 4,9 ml 1N Na₂CO₃ Lösung rührt man weitere 4 Stunden bei 70°C, entfernt das Lösungsmittel im Vakuum und nimmt in CH₂Cl₂ auf. Man extrahiert mit 2N HCl, steltt die wäßrige Phase mit 1N NaOH alkalisch und extrahiert zweimal mit CH₂Cl₂. Nach Trocknen über Na₂SO₄ wird das Lösungsmittel im Vakuumentfernt. Chromatographische Reinigung (Toluol/Essigester = 4:1) ergibt 110 mg (6.4%) schwachgelben Feststoff.
Smp.: 108°C.

### Beispiel 13

### 6-Benzyl-1-[1-(1-hydroxy-ethyl)-4-phenyl-butyl]-1,5-dihydro-pyrazolo[3,4-d]pyrimidin-4-on

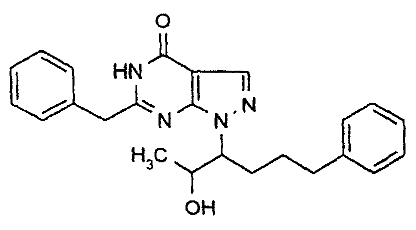

230 mg (0,76 mmol) 5-Amino-1-[1-(1-hydroxy-ethyl)-4-phenyl-butyl]-1*H*-pyrazol-4-carbonsäure-amid und 50 mg 4-Dimethylaminopyridin werden in 5 ml Pyridin gelöst und mit einer Lösung von 293 mg (1,9 mmol) Phenylacetylchlorid in 0,5 ml Toluol versetzt. Man rührt 3 Stunden bei 50°C, entfernt das Lösungsmittel im Vakuum und nimmt in 100 ml CH₂Cl₂ auf Man extrahiert mit 1N HCl, gesättigter NaHCO₃-Lösung und Wasser, trocknet über Na₂SO₄ und entfernt das Lösungsmittel im Vakuum.

Das Reaktionsprodukt wird in 8 ml Methanol und 2 ml Ethanol gelöst, mit 2 ml Wasser und 350 mg NaOH versetzt und 3 Stunden am Rückfluß erhitzt. Die Lösungsmittel werden im Vakuum entfernt, nach Zugabe von 1N HCl zweimal mit EtOAc extrahiert, über Na₂SO₄ getrocknet und das Lösungsmittel im Vakuum entfernt. Chromatographische Reinigung (PE/EE = 2:1) ergibt 101 mg (33 %) eines Diastereomerengemisches, R_{f} = 0.09 (PE/EE = 1:1) und 10 mg (3,3 %) des später eluierenden, reinen Diastereomeren.

### Beispiel 14 und Beispiel 15

### 6-(3,4-Dichloro-benzyl)-1-[1-(1-hydroxy-ethyl)-4-phenyl-butyl]-1,5-dihydro-pyrazolo[3,4-d]pyrimidin-4-on

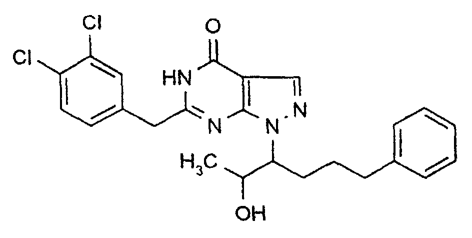

In Analogie zur Vorschrift des Beispiels 13 wird ausgehend von 400 mg (1,32 mmol) 5-Amino-1-[1-(1-Hydroxy-ethyl)-4-phenyl-butyl]-1*H*-pyrazole-4-carbonsäureamid und 735 mg (3,31 mmol) 3,4-Dichlorphenylacetylchlorid die Titelverbindung hergestellt. Dabei erhält man 65 mg (10,4 %) des schneller eluierenden Diastereoisomers, Smp.: 172°C und 63 mg (10 %) des langsamer eluierenden Diastereoisomers, Smp.: 161°C.

### Beispiel 16 und Beispiel 17

### 1-[1-(1-Hydroxy-ethyl)-4-phenyl-butyl]-6-(4-methyl-benzyl)-1,5-dihydro-pyrazolo[3,4-d]-pyrimidin-4-on

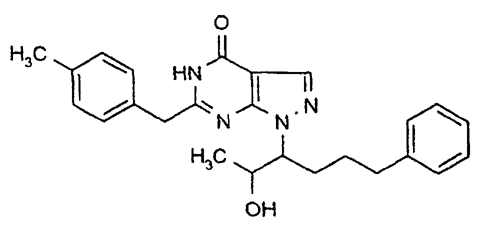

In Analogie zur Vorschrift des Beispiels 13 werden ausgehend von 400 mg (1,32 mmol) 5-Amino-1-[1-(1-Hydroxy-ethyl)-4-phenyl-butyl)-1*H*-pyrazole-4-carbonsäureamid und 578 mg (3,44 mmol) 4-Methylphenylacetylchlorid die Titelverbindungen hergestellt. Dabei erhält man 117 mg (21 %) des schneller eluierenden Diastereoisomers, Smp.; 133°C und 75 mg (13,6 %) des langsamer eluierenden Diastereoisomers, Smp.: 136°C.

### Beispiel 18

### 6-(3,4-Dimethoxy-benzyl)-1-[1-(1-hydroxy-ethyl)-4-phenyl-butyl]-1,5-dihydro-pyrazolo[3,4-d]pyrimidin-4-on

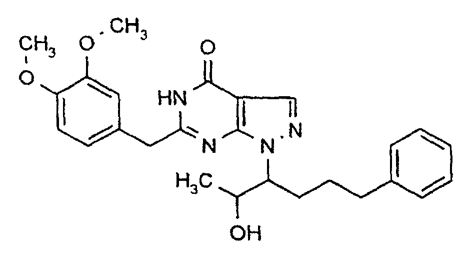

In Analogie zur Vorschrift des Beispiels 13 erhält man ausgehend von 400 mg (1,32 mmol) 5-Amino-1-[1-(1-Hydroxy-ethyl)-4-phenyl-butyl]-1*H*-pyrazol-4-carbonsäure-amid und 711 mg (3,32 mmol) 3,4-Dimethoxyphenylacetylchlorid die Titelverbindung.
Ausbeute: 303 mg (50 %).
Smp.: 85°C.

### Beispiel 19 und Beispiel 20

### 6-(4-Fluor-benzyl)-1-[1-(1-hydroxy-ethyl)-4-phenyl-butyl]-1,5-dihydro-pyrazolo[3,4-d]-pyrimidin-4-one

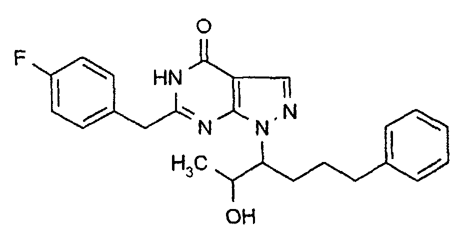

In Analogie zur Vorschrift des Beispiels 13 werden ausgehend von 400 mg (1,32 mmol) 5-Amino-1-[1-(1-Hydroxy-ethyl)-4-phenyl-butyl]-1*H*-pyrazol-4-carbonsäure-amid und 570 mg (3,31 mmol) 4-Fluorphenylacetylchlorid die Titelverbindungen hergestellt. Dabei erhält man 143 mg (27 %) des schneller eluierenden Diastereoisomers, Smp.: 103°C und 111 mg (21 %) des langsamer eluierenden Diastereoisomers, Smp.: 107°C.

### Beispiel 21 und Beispiel 22

### 6-(4-Chlor-benzyl)-1-[1-(1-hydroxy-ethyl)-4-phenyl-butyl]-1,5-dihydro-pyrazolo[3,4-d]-pyrimidin-4-on

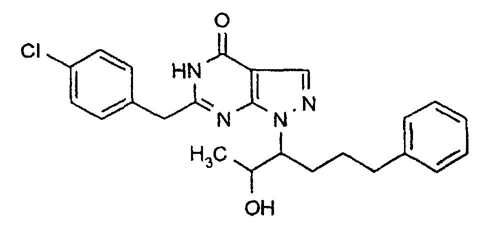

In Analogie zur Vorschrift des Beispiels 13 werden ausgehend von 400 mg (1,32 mmol) 5-Amino-1-[1-(1-Hydroxy-ethyl)-4-phenyl-butyl]-1*H*-pyrazol-4-carbonsäure-amid und 626 mg (3,77 mmol) 4-Chlorphenylacetylchlorid die Titelverbindungen hergestellt. Dabei erhält man 150 mg (26 %) des schneller eluierenden Diastereoisomers, Smp.: 125°C und 90 mg (16 %) des langsamer eluierenden Diastereoisomers, Smp.: 101°C.

### Beispiel 23 und Beispiel 24

### 1-[1-(1-Hydroxy-ethyl)-4-phenyl-butyl]-6-(3-methoxy-benzyl)-1,5-dihydro-pyrazolo[3,4-d]pyrimidin-4-on

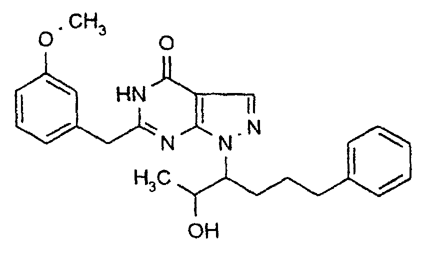

In Analogie zur Vorschrift des Beispiels 13 werden ausgehend von 400 mg (1,32 mmol) 5-Amino-1-[1-(1-Hydroxy-ethyl)-4-phenyl-butyl]-1*H*-pyrazol-4-carbonsäure-amid und 610 mg (3,81 mmol) 3-Methoxyphenylacetylchlorid die Titelverbindungen hergestellt. Dabei erhält man 160 mg (28 %) des schneller eluierenden Diastereoisomers, Smp.: 92°C und 145 mg (25%) des langsamer eluierenden Diastereoisomers, Smp.: 54°C.

### Beispiel 25 und Beispiel 26

### 6-Biphenyl-4-yl-methyl-1-[1-(1-hydroxy-ethyl)-4-phenyl-butyl]-1,5-dihydro-pyrazolo[3,4-d]pyrimidin-4-on

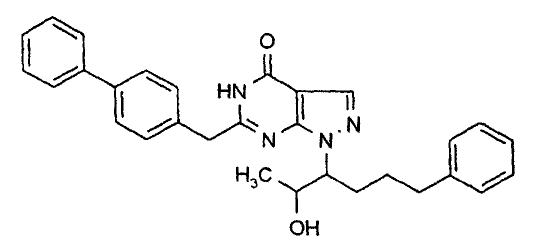

In Analogie zur Vorschrift des Beispiels 13 werden ausgehend von 954 mg (3,16 mmol) 5-Amino-1-[1-(1-Hydroxy-ethyl)-4-phenyl-butyl]-1*H*-pyrazole-4-carbon-säure-amid und 1,82 g (7,91 mmol) 4-Phenylphenylacetylchlorid die Titelverbindungen hergestellt. Dabei erhält man 410 mg (27 %) des schneller eluierenden Diastereoisomers, Smp.: 90°C und 160 mg (11 %) des langsamer eluierenden Diastereoisomers, Smp.: 142°C.

### Beispiel 27 und Beispiel 28

### 1-[1-(1-Hydroxy-ethyl)-4-phenyl-butyl]-6-(4-methoxy-benzyl)-1,5-dihydro-pyrazolo[3,4-d]pyrimidin-4-on

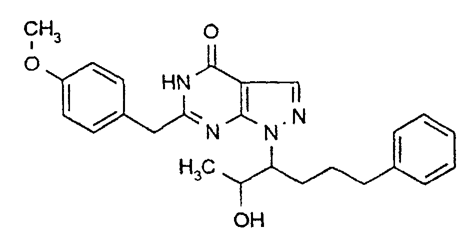

In Analogie zur Vorschrift des Beispiels 13 werden ausgehend von 1 g (3,31 mmol) 5-Amino-1-[1-(1-Hydroxy-ethyl)-4-phenyl-butyl]-1*H*-pyrazol-4-carbonsäure-amid und 1,52 g (8,26 mmol) 4-Methoxyphenylacetylchlorid die Titelverbindungen hergestellt. Dabei erhält man 240 mg (17%) des schneller eluierenden Diastereoisomers, Smp.: 41°C und 134 mg (9 %) des langsamer eluierenden Diastereoisomers, Smp.: 48°C.

### Beispiel 29 und Beispiel 30

### 6-(4-Brom-benzyl)-1-[1-(1-hydroxy-ethyl)-4-phenyl-butyl]-1,5-dihydro-pyrazolo[3,4-d]-pyrimidin-4-on

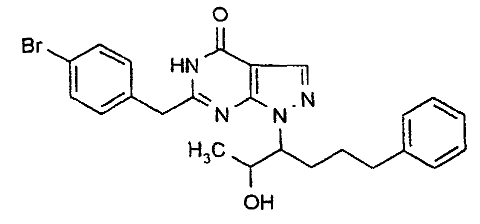

In Analogie zur Vorschrift des Beispiels 13 werden ausgehend von 2,1g (6,95 mmol) 5-Amino-1-[1-(1-Hydroxy-ethyl)-4-phenyl-butyl]-1*H*-pyrazol-4-carbonsäure-amid und 4,05 g (17,38 mmol) 4-Bromphenylacetylchlorid die Titelverbindungen hergestellt. Dabei erhält man 594 mg (18 %) des schneller eluierenden Diastereoisomers, Smp.: 117°C und 372 mg (11 %) des langsamer eluierenden Diastereoisomers, Smp.: 116°C.

### Beispiel 31

### 1-[1-(1-Hydroxy-ethyl)-4-phenyl-butyl]-6-(hydroxy-phenyl-methyl)-1,5-dihydro-pyrazolo[3,4-d]pyrimidin-4-on

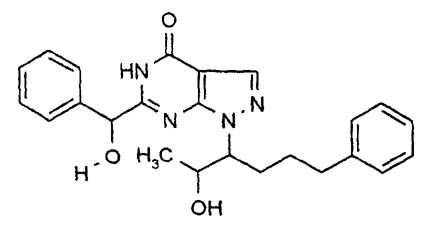

Ausgehend von 1,25 g (4,14 mmol) 5-Amino-1-[1-(1-Hydroxy-ethyl)-4-phenyl-butyl]-1*H*pyrazol-4-carbonsäure-amid und 2,20 g (10,38 mmol) D,L-Acetylmandelsäurechlorid erhält man die Titelverbindung mit 72 mg (4,2 %) Ausbeute.
R_{f}= 0.10 (Cyclohexan/Essigester = 2:1).

### Beispiel 32 und Beispiel 33

### 6-Benzyl-1-[1-(1-hydroxy-ethyl)-4-phenyl-butyl]-3-methyl-1,5-dihydro-pyrazolo[3,4-d]-pyrimidin-4-on

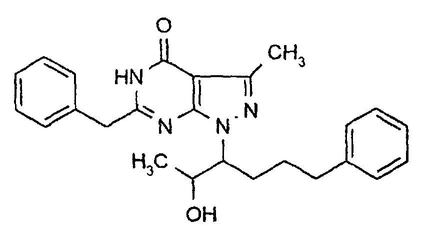

805 mg (2,55 mmol) 5-Amino-1-[1-(1-hydroxy-ethyl)-4-phenyl-butyl]-3-methyl-1*H*-pyrazol-4-carbonsäure-amid und 140 mg 4-Dimethylaminopyridin werden in 20 ml Pyridin gelöst und mit einer Lösung von 991 mg (6,48 mmol) Phenylacetylchlorid in 1 ml Toluol versetzt. Man rührt 3 Stunden bei 50°C, entfernt das Lösungsmittel im Vakuum und nimmt in 100 ml CH₂Cl₂ auf. Man extrahiert mit 1N HCl, gesättigter NaHCO₃-Lösung und Wasser, trocknet über Na₂SO₄ und entfernt das Lösungsmittel im Vakuum.

Das Reaktionsprodukt wird in 45 ml Ethanol gelöst, mit 8 ml Wasser und 2.0g NaOH versetzt und 3 Stunden am Rückfluß erhitzt. Die Lösungsmittel werden im Vakuum entfernt, nach Zugabe von 1N HCI zweimal mit EtOAc extrahiert, über Na₂SO₄ getrocknet und das Lösungsmittel im Vakuum entfernt. Chromatographische Reinigung (PE/EE= 2:1) ergibt 216 mg (20 %) des schneller eluierenden Diastereoisomers, R_{f} = 0.2 (Cyclohexan/Essigester = 2:1) und 156 mg (15 %) des langsamer eluierenden Diastereoisomers, R_{f}= 0.1 (Cyclohexan/Essigester = 2:1).

### Beispiel 34 und Beispiel 35

### 6-(3,4-Dichlor-benzyl)-1-[1-(1-hydroxy-ethyl)-4-phenyl-butyl]-3-methyl-1,5-dihydropyrazolo[3,4-d]pyrimidin-4-on

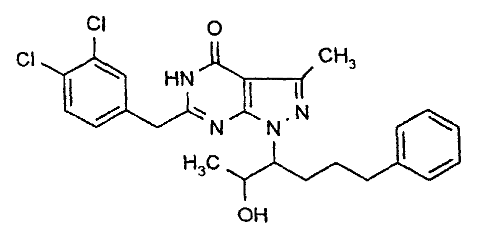

In Analogie zur Vorschrift der Beispiele 32 und 33 werden ausgehend von 805 mg (2,55 mmol) 5-Amino-1-[1-(1-hydroxy-ethyl)-4-phenyl-butyl]-3-methyl-1*H*-pyrazol-4-carbonsäure-amid und 1.41g (6.59 mmol) 3,4-Dichlorphenylacetylchlorid eingestzt. Dabei erhält man 217 mg (18 %) des schneller eluierenden Diastereoisomers, R_{f} = 0.2 (Cyclohexan/Essigester = 2:1) und 186 mg (15 %) des langsamer eluierenden Diastereoisomers, Smp.: 160°C.

### Beispiel 36

### 6-(3,4-Dimethoxy-benzyl)-1-[1-(1-hydroxy-ethyl)-4-phenyl-butyl]-3-methyl-1,5-dihydropyrazolo[3,4-d]pyrimidin-4-on

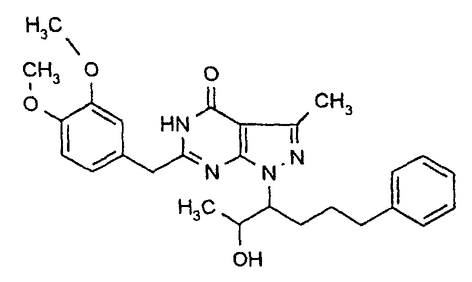

Die Titelverbindung erhält man ausgehend von 805 mg (2,55 mmol) 5-Amino-1-[1-(1-hydroxy-ethyl)-4-phenyl-butyl]-3-methyl-1*H*-pyrazol-4-carbonsäure-amid und 1,37 g (6,40 mmol) 3,4-Dimethoxyphenylacetylchlorid.
Ausbeute: 139 mg (9 %).
R_{f}= 0.1 (Cyclohexan/Essigester = 2:1).

### Beispiel 37

### 1-(1-Acetyl-4-phenyl-butyl)-6-(4-methoxy-benzyl)-1,5-dihydro-pyrazolo[3,4-d]pyrimidin-4-on

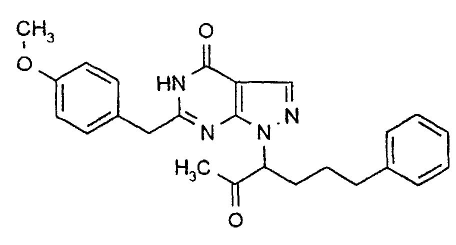

151 mg (0,35 mmol) 1-[1-(1-Hydroxy-ethyl)-4-phenyl-butyl]-6-(4-methoxy-benzyl)-1,5-dihydro-pyrazolo[3,4-*d*]pyrimidin-4-on werden in 1 ml DMSO und 3,5 ml Dichlormethan gelöst und auf 0°C abgekühlt. Die Lösung wird mit 0,485 ml Triethylamin und 245 mg SO₃-Pyridin Komplex versetzt und 15 Stunden bei Raumtemperatur gerührt. Es wird mit 20 ml Dichlormethan verdünnt, mit 1N HCl und gesättigter NaHCO₃ Lösung extrahiert, über Na₂SO₄ getrocknet und das Lösungsmittel im Vakuum entfernt. Chromatographische Reinigung (PE/EE = 2:1) ergibt 115 mg (76 %), Smp.:129°C.

### Beispiel 38

### 1-(1-Acetyl-4-phenyl-butyl)-6-biphenyl-4-ylmethyl-1,5-dihydro-pyrazolo[3,4-d]pyrimidin-4-on

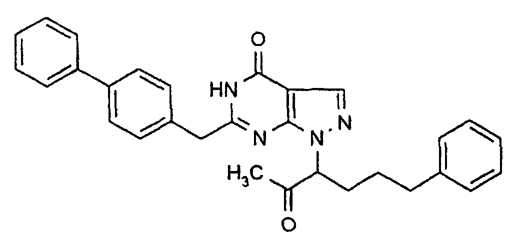

Die Titlverbindung erhält man ausgehend von 299 mg (0,62 mmol) 6-Biphenyl-4-ylmethyl-1-[1-(1-hydroxy-ethyl)-4-phenyl-butyl]-1,5-dihydro-pyrazolo[3,4-*d*]-pyrimidin-4-on mit einer Ausbeute von 268 mg (91 %).
Smp.: 107°C.

### Beispiel 39

### 1-(1-Acetyl-4-phenyl-butyl)-6-(3,4-dimethoxy-benzyl)-3-methyl-1,5-dihydro-pyrazolo[3,4-d]pyrimidin-4-on

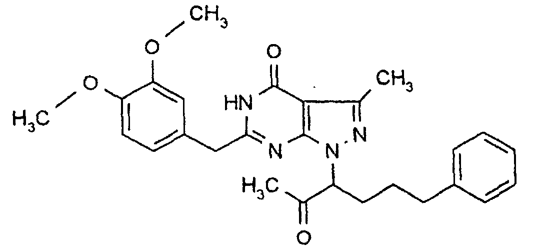

Die Titelverbindung erhält man ausgehend von 237 mg (0,51 mmol) 6-(3,4-Dimethoxybenzyl)-1-[1-(1-hydroxy-ethyl)-4-phenyl-butyl]-3-methyl-1,5-dihydro-pyrazolo[3,4-*d*]pyrimidin-4-on mit einer Ausbeute von 165 mg (70 %).
Smp.: 120°C.

### Beispiel 40

### 6-[4-(morpholine-4-sulfonyl)-benzyl][1-(1-hydroxy-ethyl)-4-phenyl-butyl]-3-methyl-1,5-dihydro-pyrazolo[3,4-d]pyrimidin-4-on

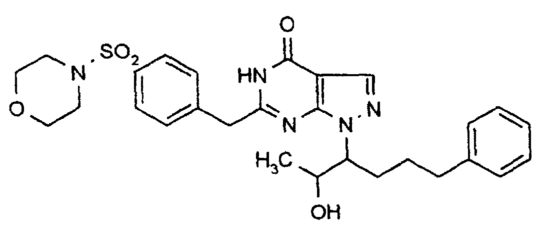

785 mg (2,48 mmol) 5-Amino-1-[1-(1-hydroxy-ethyl)-4-phenyl-butyl]-3-methyl-1*H*-pyrazol-4-carbonsäure-amid, 1,40 g Kalium-*tert*.-butylat und 1,67 g (5,58 mmol) 4-(Morpholinosulfonyl)-phenylessigsäuremethylester werden in 18 ml Ethanol über Nacht refluxiert. Das Lösungsmittel wird im Vakuum entfernt, in Dichlormethan aufgenommen, mit 1N HCl extrahiert, die organische Phase über Na₂SO₄ getrocknet und einrotiert. Zweimalige chromatographische Reinigung (Dichlormethan/Methanol = 30/1) ergibt 375 mg (28 %).
R_{f}= 0.33 (Dichlormethan/Methanol = 30/1).

### Beispiel 41

### 1-(1-Acetyl-4-phenyl-butyl)-6-(4-(3-pyridyl)-benzyl)-1,5-dihydro-pyrazolo[3,4-d]pyrimidin-4-on

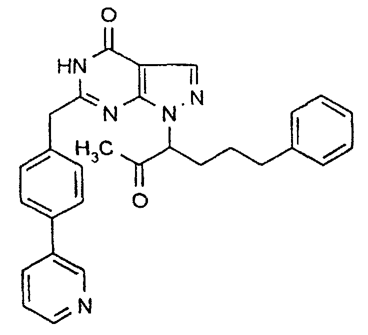

Zu einer Lösung von 177 mg (0,37 mmol) 1-(1-Acetyl-4-phenyl-butyl)6-(4-brom-benzyl)-1,5-dihydro-pyrazolo[3,4-*d*]pyrimidin-4-on in 4 ml THF gibt man unter Argon 71 mg (0,48 mmol) Diethyl-(3-pyridyl)-boran und 16 mg Tetrakistriphenylphosphin-palladium und rührt eine Stunde bei 70°C. Nach Zugabe von 0,522 ml 2N Na₂CO₃ Lösung rührt man weitere 4 Stunden bei 70°C, gibt weitere 27 mg (0,185 mmol) Diethyl-(3-pyridyl)-boran und 16 mg Tetrakistriphenylphosphin-palladium zu und rührt 14 Stunden unter Rückfluß. Das Lösungsmittel wird im Vakuum entfernt und der Rückstand in CH₂Cl₂ aufgenommen. Man extrahiert mit 2N HCl, stellt die wäßrige Phase mit 1N NaOH alkalisch und extrahiert zweimal mit CH₂Cl₂. Nach Trocknen über Na₂SO₄ wird das Lösungsmittel im Vakuum entfernt. Chromatographische Reinigung (Toluol/Essigester = 4:1) ergibt 110 mg (6,4 %) schwachgelber Feststoff.
Smp.: 108°C.

### Beispiel 42

### 6-Benzyl-3-methoxypyrazolo[3,4-d]pyrimidin-4(5H)-on

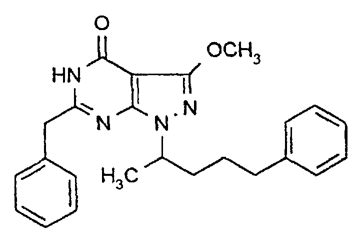

4,6 g 5-Amino-4-cyano-3-methoxy-1-[(5-phenyl)-pent-2-yl]-pyrazol werden in 25 ml Pyridin gelöst. Bei ca. 0°C werden 2,2 ml Phenylessigsäurechlorid langsam zugetropft. Die Reaktionsmischung wird über Nacht bei ca. 20°C gerührt. Dann werden noch 0,7 ml Phenylessigsäurechlorid nachgegeben und 2 h nachgerührt. Zur Aufarbeitung wird mit Ethylacetat verdünnt und zweimal mit verdünnter Zitronensäurelösung extrahiert. Die Ethylacetatphase wird mit Magnesiumsulfat getrocknet und i.V. eingedampft. Man erhält so 7,5 g braunes Öl, das dann in einer Mischung aus 75 ml 1n Natronlauge und 4,5 ml 35% igem Wasserstoffperoxid 6 h auf 100°C erhitzt wird. Zur Aufarbeitung wird mit Eis versetzt und zweimal mit Ethylacetat extrahiert. Die vereinigten Ethylacetatphasen werden mit verd. Thiosulfatlösung und mit verd. Zitronensäurelösung gewaschen, mit Magnesiumsulfat getrochnet und i.V. eingedampft. Man erhält 5,1 g gelbes Öl, das an Kieselgel (Merck Si 60 0,04-0,063 mm) mit einem Cyclohexan/Ethylacetat-Gemisch im Verhältnis von 100:0 bis 1:1 chromatographiert wird. Man erhält 5 Fraktionen:

| | |
|---|---|
| 1 Fraktion | 0,46 g 4-Cyano-3-methoxy-5-(phenylacetyl)amino-1-[5-phenyl-pent-2-yl]pyrazol |
| 2. Fraktion | 0,08 g 5-Amino-4-cyano-3-methoxy-1-[(5-phenyl)-pent-2-yl]-pyrazol |
| 3. Fraktion | 2,0 g 6-Benzyl-3-methoxy-1-[5-phenyl-pent-2-yl]pyrazolo[3,4-d]-pyrimidin-4(5H)-on verunreinigt mit 5-Amino-4-cyano-3-methoxy-1-[5-phenyl-pent-2-yl]pyrazol |
| 4. Fraktion | 0,9 g 6-Benzyl-3-methoxy-1-[5-phenyl-pent-2-yl]pyrazolo[3,4-d]-pyrimidin-4(5H)-on |
| 5.Fraktion | 0,5 g 5-Amino-4-carboxamido-3-methoxy-1-[5-phenyl-pent-2-yl]-pyrazol |

NMR von 6-Benzyl-3-methoxy-1-[5-phenyl-pent-2yl]-pyrazolo[3,4-d]pyrimidin-4(5H)-on (4.Fraktion)
(300 MHZ, CD₃OD) 1,2-1,5[2]m; 1,4[3]d J=8Hz; 1,6-1,75[1]m; 1,9-2,05[1]m; 2,-2,6[2]m; 3,9[2]s; 3,95[3]s; 4,75-4,85[1]m; 7,0[2]d; 7,05-7,35[8]m
DC von 6-Benzyl-3-methoxy-1-[5-phenyl-pent-2yl]-pyrazolo[3,4-d]pyrimidin-4(5H)-on (4.Fraktion)
R_{f}-Wert = 0,4 ; Laufmittel: Petrolether/Ethylacetat 1:1; Merck Si60 Art.Nr. 1.05719
NMR von 5-Amino-4-carboxamido-3-methoxy-1-[5-phenyl-pent-2-yl]-pyrazol (5, Fraktion)
(300 MHZ, CD₃OD) 1,3[3]d J=8Hz; 1,35-1,65[3]m; 1,85-2,0[1]m; 2,5-2,65[2]m; 3,9[3]s; 4,1-4,2[1]m; 7,1-7,15[3]m; 7,2-7,25[2]m
DC von 5-Amino-4-carboxamido-3-methoxy-1-[5-phenyl-pent-2-yl]-pyrazol (5.Fraktion) R_{f}-Wert = 0,16 ; Laufmittel: Petroether/Ethylacetat 1:1; Merck Si60 Art.Nr. 1.05719

### Beispiel 43

### 6-Benzyl-1-[5-phenyl-pent-2yl]-pyrazolo[3,4-d]pyrimidin-3,4(2H,5H)-on

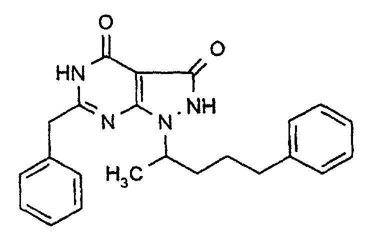

1,6 g 6-Benzyl-3-methoxy-1-[5-phenyl-pent-2yl]-pyrazolo[3,4-d]pyrimidin-4(5H)-on (3. Fraktion von obigem Beispiel) werden zusammen mit 0,75 g Natriumjodid in 25 ml absolutem Acetonitril gelöst. Nach Zugabe von 0,63 ml Trimethylchlorsilan wird 2 h zum Rückfluß erhitzt. Zur Aufarbeitung wird die Reaktionsmischung in verd. Natriumthiosulfatlösung gegossen und mit Ethylacetat extrahiert. Das zweiphasige Gemisch enthält das Produkt als weißen, kristallinen Niederschlag. Der Niederschlag wird abgesaugt, mit Ethylacetat und Wasser gewaschen und i.V. getrocknet. Man erhält so 0,636 g (= 61 % d.Th.) 6-Benzyl-1-[5-phenyl-pent-2-yl]-pyrazolo[3,4-d]pyrimidin-3,4(2H,5H)-on, Fp = 275°C.
NMR von 6-Benzyl-1-[5-phenyl-pent-2yl]-pyrazolo[3,4-d]pyrimidin-3,4(2H,5H)-on (200 MHZ, DMSO-d6) 1,1-1,4[2]m; 1,3[3]d J=8Hz; 1,55-1,9[2]m; 2,35-2,6[2]m; 3,85[2]s; 4,6-4,8[1]m; 7,0-7,4[10]m; 10,9[1]s breit; 11,9[1]s breit

### Beispiel 44

### 6-(3,4-Dimethoxybenzyl)-3-methoxy-1-[5-phenyl-pent-2-yl]-pyrazolo[3,4-d]pyrimidin-4(5H)-on

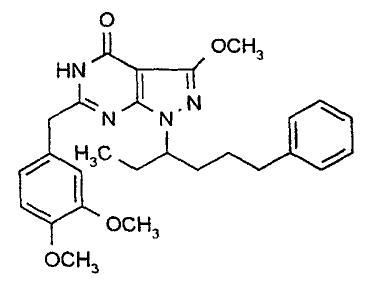

0,416 g 5-Amino-4-carboxamido-3-methoxy-1-[5-phenyl-pent-2-yl]-pyrazol werden zusammen mit 1,34 g 3,4-Dimethoxyphenylessigsäureethylester und 1,18 g Kalium tert.butylat in 6 ml absolutem Ethanol 2 h zum Rückfluß erhitzt. Zur Aufarbeitung wird mit Ethylacetat verdünnt und zweimal mit ges. Natriumhydrogencarbonatlösung gewaschen. Die Ethylacetatphase wird mit Magnesiumsulfat getrocknet und i.V. eingedampft. Der Eindampfrückstand kristallisiert aus Diethylether. Nach Absaugen und Trocknen i.V. erhält man 0,528 g (= 82,7 % d.Th.) 6-(3,4-Dimethoxybenzyl)-3-methoxy-1-[5-phenylpent-2-yl]-pyrazolo[3,4-d]pyrimidin-4(5H)-on als weiße Kristalle, Fp = 148°C.
NMR (200 MHZ, CDCl₃) 1,45[3]d J=8Hz, 1,35-1,55[2]m; 1,65-1,85[1]m; 1,95-2,15[1]m; 2,5-2,7[2]m; 3,8[3]s; 3,85[3]s; 3,95[2]s; 4,0[3]s; 4,75-4,90[1]m; 6,75[1]d J=8Hz;6,85-7,0[2]m; 7,1-7,3[5]m
DC R_{f}-Wert = 0,5; Laufmittel: Dichlormethan/Methanol 10:1; Merck Si60 Art.Nr. 1.05719

### Beispiel 45

### 6-Benzyl-3-methoxy-1-[2-hydroxy-(6-phenyl)-hex-3-yl]-pyrazolo[3,4-d]pyrimidin-4(5H)-on

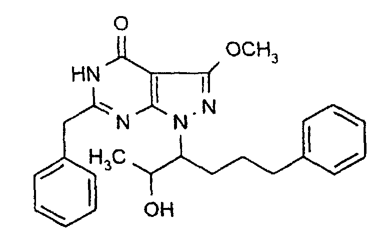

0,94 g 5-Amino-4-cyano-3-methoxy-1-[2-hydroxy-6-phenyl-hex-3-yl]-pyrazol werden in 5 ml Pyridin gelöst. Bei ca. 0°C werden 0,98 ml Phenylessigsäurechlorid langsam zugetropft. Die Reaktionsmischung wird über Nacht bei ca. 20°C gerührt. Dann werden noch 0,25 ml Phenylessigsäurechlorid nachgegeben und 1 h nachgerührt. Zur Aufarbeitung wird mit Ethylacetat verdünnt und zweimal mit verdünnter Zitronensäurelösung extrahiert. Die Ethylacetatphase wird mit Magnesiumsulfat getrocknet und i.V. eingedampft. Man erhält so 2,16 g braunes Öl, das dann in einer Mischung aus 15 ml in Natronlauge und 0,78 ml 35% igem Wasserstoffperoxid 6 h auf 100°C erhitzt wird. Zur Aufarbeitung wird mit Eis versetzt und zweimal mit Ethylacetat extrahiert. Die vereinigten Ethylacetatphasen werden mit verd. Thiosulfatlösung und mit verd. Zitronensäurelösung gewaschen, mit Magnesiumsulfat getrochnet und i.V. eingedampft. Man erhält 2 g Öl, das an Kieselgel (Merck Si60 0,04-0,063 mm) mit einem Cyclohexan/Ethylacetat-Gernisch im Verhältnis von 50:0 bis 2:1 chromatographiert wird. Man erhält so mehrere Fraktionen, von denen eine nach Eindampfen 0,185 g (= 15 % d.Th.) 6-Benzyl-3-methoxy-1-[2-hydroxy-(6-phenyl)-hex-3-yl ]-pyrazolo[3,4-d]pyrimidin-4(5H)-on als Diastereomerengemisch ergibt.
NMR (300 MHZ, CD₃OD) 0,9 und 1,1[3]d J=8 Hz; 1,2-1,4[2]m; 1,65-1,8 und 1,95-2,1[2]m; 2,4-2,7[2]m; 3,9 [3]s; 3,95[2]s; 4,0-4,1[1]m; 4,4-4,55[1]m; 6,95-7,35[10]m.

### Beispiel 46

### 6-(3,4-Methylendioxy-benzyl)-1-(1-methyl-4-phenyl-butyl)-1,5-dihydro-pyrazolo[3,4-d]pyrimidin-4-on

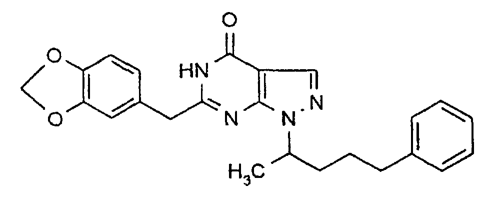

18 mg (0,066 mmol) 5-Amino-1-(1-methyl-4-phenyl-butyl)-1*H*-pyrazol-4-carbonsäureamid und 41 mg (0,21 mmol) 3,4-Methylendioxyphenylessigsäuremethylester werden in 1 ml einer 0,5M ethanolischen Kalium-*tert*.-butylatlösung 6 Stunden am Rückfluß erhitzt. Nach Zugabe von Dichlormethan und gesättigter wäßriger Natriumhydrogencarbonatlösung werden die Phasen getrennt. Chromatographische Reinigung ergibt 18 mg (64 %) eines Feststoffs, R_{f}= 0.34 (Dichlormethan/Methanol = 15:1).

### Beispiel 47

### 6-(3,4,5-Trimethoxy-benzyl)-1-(1-methyl-4-phenyl-butyl)-3-ethyl-1,5-dihydro-pyrazolo[3,4-d]pyrimidin-4-on

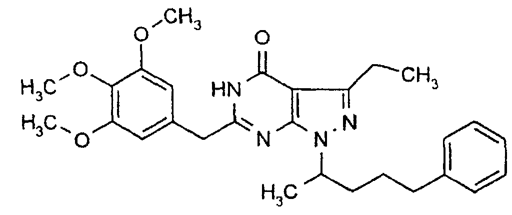

400 mg (1,4 mmol) 5-Amino-3-ethyl-1-(1-methyl-4-phenyl-butyl)-1*H*-pyrazol-4-carbonsäure-amid, 750 mg (6,7 mmol) Kalium-*tert*.-butanolat und 720 mg (3,0 mmol) 3,4,5-Trimethoxy-phenylessigsäuremethylester werden in 10 ml Ethanol 16 Stunden am Rückfluß erhitzt. Nach Entfernen des Lösungsmittels wird mit 1N HCl sauer gestellt, 3 mal mit Dichlormethan extrahiert, die org. Phase über Natriumsulfat getrocknet und das Lösungsmittel im Vakuum entfernt. Chromatographische Reinigung ergibt 286 mg (40 %) eines Feststoffs, R_{f}= 0.62 (Ethylacetat/Cyclohexan = 2:1).

### Beispiel 48

### 6-(3,4-Methylendioxy-benzyl)-1-(1-methyl-4-phenyl-butyl)-3-ethyl-1,5-dihydro-pyrazolo[3,4-d]pyrimidin-4-on

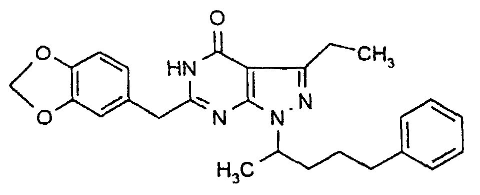

19 mg (0,063 mmol) 5-Amino-1-(1-methyl-4-phenyl-butyl)-3-ethyl-1*H*-pyrazol-4-carbonsäureamid und 35 mg (0,180 mmol) 3,4-Methylendioxyphenylessigsäuremethylester werden in 0,9 ml einer 0,5M ethanolischen Kalium-*tert*.-butylatlösung 6 Stunden am Rückfluß erhitzt. Nach Zugabe von Dichlormethan und gesättigter wäßriger Natriumhydrogencarbonatlösung werden die Phasen getrennt. Chromatographische Reinigung ergibt 14 mg (48 %) eines Feststoffs, R_{f} = 0.64 (Dichlormethan/Methanol = 15:1).

### Beispiele 49 und 50

### 6-(3,4-Dimethoxy-benzyl)-1-[1-(1-hydroxy-ethyl)-4-phenyl-butyl]-3-ethyl-1,5-dihydro-pyrazolo[3,4-d]pyrimidin-4-one

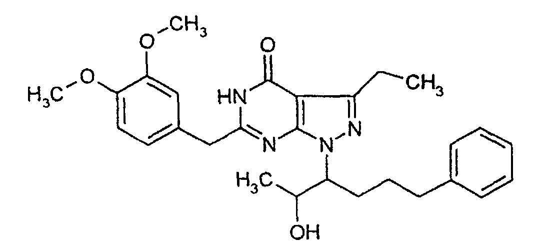

1,0 g (3,03 mmol) 5-Amino-1-[1-(1-hydroxy-ethyl)-4-phenyl-butyl]-3-ethyl-1*H*pyrazol-4-carbonsäureamid, 1,60 g Kalium-*tert*.-butylat und 1,40 g (19 mmol) 3,4-Dimethoxyphenylessigsäuremethylester werden in 20 ml Ethanol über Nacht refluxiert. Das Lösungsmittel wird im Vakuum entfernt, der Rückstand in Dichlormethan aufgenommen, mit 1N HCl extrahiert, die organische Phase über Na₂SO₄ getrocknet und einrotiert. Chromatographische Reinigung (Cyclohexan/EE = 2:1, 1% Ameisensäure) und chromatographische Trennung der Diastereomere ergibt 232 mg (16 %) des schneller eluierenden Diastereomeren, R_{f}= 0.2 (Cyclohexan/Essigester = 2:1) und 150 mg (10 %) des langsamer eluierenden Diastereomeren, R_{f} = 0.15 (Cyclohexan/Essigester = 2:1).

### Beispiel 51

### 6-(3,4-Methylendioxy-benzyl)-1-[1-(1-hydroxy-ethyl)-4-phenyl-butyl]-3-ethyl-1,5-dihydro-pyrazolo[3,4-d]pyrimidin-4-on

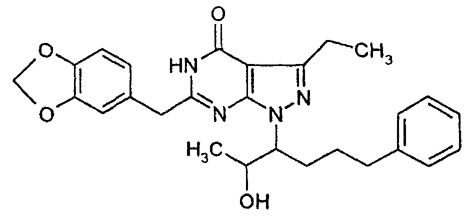

10 mg (0,037 mmol) 5-Amino-1-[1-(1-hydroxy-ethyl)-4-phenyl-butyl]-3-ethyl-1*H*pyrazol-4-carbonsäureamid und 30 mg (0,129 mmol) 3,4-Methylendioxyphenylessigsäuremethylester werden in 0,3 ml einer 0,5M ethanolischen Kalium-*tert*.butylatlösung 6 Stunden am Rückfluß erhitzt. Nach Zugabe von Dichlormethan und gesättigter wäßriger Natriumhydrogencarbonatlösung werden die Phasen getrennt. Chromatographische Reinigung ergibt 3 mg (20 %) eines Feststoffs, R_{f} = 0.65 (Dichlormethan/Methanol = 15:1).

### Beispiel 52

### 6-(3,4-Methylendioxy-benzyl)-1-[1-(1-hydroxy-ethyl)-4-phenyl-butyl]-1,5-dihydropyrazolo[3,4-d]pyrimidin-4-on

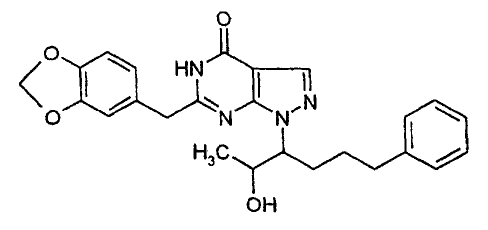

20 mg (0,066 mmol) 5-Amino-1-[1-(1-hydroxy-ethyl)-4-phenyl-butyl]-1*H*-pyrazol-4-carbonsäureamid und 41 mg (0,210 mmol) 3,4-Methylendioxyphenylessigsäuremethylester werden in 1,06 ml einer 0,5M ethanolischen Kalium-*tert*.-butylatlösung 6 Stunden am Rückfluß erhitzt. Nach Zugabe von Dichlormethan und gesättigter wäßriger Natriumhydrogencarbonatlösung werden die Phasen getrennt. Chromatographische Reinigung ergibt 19 mg (64 %) eines Feststoffs, R_{f} = 0.20 (Dichlormethan/Methanol= 15:1).

### Beispiel 53

### 6-(3,4-Methylendioxy-benzyl)-1-[1-(1-hydroxy-ethyl)-4-phenyl-butyl]-3-methyl-1,5-dihydro-pyrazolo[3,4-d]pyrimidin-4-on

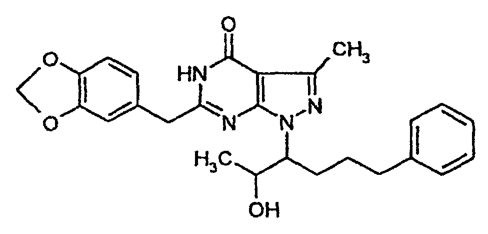

10 mg (0,037 mmol) 5-Amino-1-[1-(1-hydroxy-ethyl)-4-phenyl-butyl]-3-methyl-1*H*pyrazol-4-carbonsäureamid und 30 mg (0,129 mmol) 3,4-Methylendioxyphenylessigsäuremethylester werden in 0,3 ml einer 0,5M ethanolischen Kalium-*tert.*butylatlösung 6 Stunden am Rückfluß erhitzt. Nach Zugabe von Dichlormethan und gesättigter wäßriger Natriumhydrogencarbonatlösung werden die Phasen getrennt. Chromatographische Reinigung ergibt 5 mg (31 %) eines Feststoffs, R_{f} = 0.44 (Dichlormethan/Methanol = 15:1).

### Beispiele 82 und 83

### 6-Benzyl-3-ethyl-1-[2-hydroxy-(6-phenyl)-hex-3-yl]-pyrazolo[3,4-d]pyrimidin-4(5H)-on

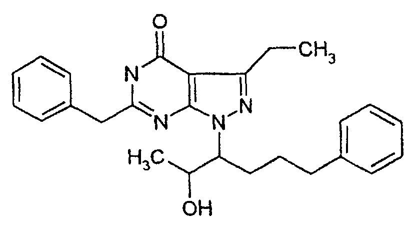

6 mg (0,02 mmol) 5-Amino-4-acetamido-3-ethyl-1-[2-hydroxy-6-phenyl-hex-3-yl]-pyrazol (Diastereomerengemisch) werden zusammen mit ca. 20 mg (0,08 mmol) Phenylessigester und 0,2 ml (0,1 mmol 0,5 molarer NaOEt in EtOH 1,5 h unter Argon am Rückfluß erhitzt. Der Ansatz wird mit 0,5 ml Dichlormethan und 0,5 ml 10 % Natriumhydrogencarbonatlösung versetzt und kräftig gerührt. Die organische Phase wird abgetrennt und durch Chromatographie an Kieselgel gereinigt. Man erhält so zwei Fraktionen:
0,5 mg (= 5,8 % d.Th.) unpolareres Diastereomer von 6-Benzyl-3-ethyl-1-[2-hydroxy-(6-phenyl)-hex-3-yl]-pyrazolo[3,4-d]pyrimidin-4(5H)-on
(DC R_{f}-Wert = 0,75; Laufmittel: Dichlormethan/Methanol 10:1; Merck Si60 Art.-Nr. 1.05719) und
0,3 mg (= 3,5 % d.Th.) polareres Diastereomer von 6-Benzyl-3-ethyl-1-[2-hydroxy-(6-phenyl)-hex-3-yl]-pyrazolo[3,4-d]pyrimidin-4(5H)-on
(DC R_{f}-Wert = 0.57; Laufmittel: Dichlormethan/Methanol 10:1; Merck Si60 Art.-Nr. 1.05719).

### Beispiel 84

### 6-(4-Aminobenzyl)-3-ethyl-1-[2-hydroxy-(6-phenyl)-hex-3-yl]pyrazolo[3,4-d]pyrimidin-4(5H)-on

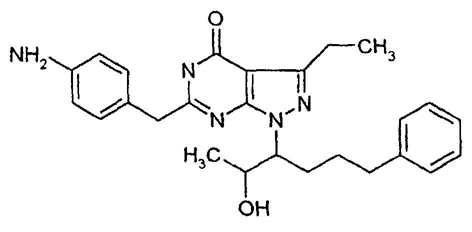

6 mg (0,02 mmol) 5-Amino-4-acetamido-3-ethyl-1-[2-hydroxy-6-phenyl-hex-3-yl]-pyrazol (Diastereomerengemisch) werden zusammen mit ca. 20 mg (0,08 mmol) 4-Aminophenylessigester und 0,2 ml (0,1 mmol) 0,5 molarer NaOEt in EtOH 1,5 h unter Argon am Rückfluß erhitzt. Der Ansatz wird mit 0,5 ml Dichlormethan und 0,5 ml 10 % Natriumhydrogencarbonatlösung versetzt und kräftig gerührt. Die organische Phase wird abgetrennt und durch Chromatographie an Kieselgel gereinigt. Man erhält so eine saubere Fraktion:
1.6 mg (= 18 % d.Th.) 6-(4-Aminobenzyl)-3-ethyl-1-[2-hydroxy-(6-phenyl)-hex-3-yl]-pyrazolo[3,4-*d*]pyrimidin-4(5H)on (Diastereomerengemisch)
(DC R_{f}-Wert = 0.49; Laufmittel: Dichlormethan/Methanol 10:1; Merck Si60 Art.-Nr. 1.05719).

### Beispiel 85

### 6-(4-Morpholinosulfonylbenzyl)-3-ethyl-1-[2-hydroxy-(6-phenyl)-hex-3-yl]-pyrazolo[3,4-d]pyrimidin-4(5H)-on

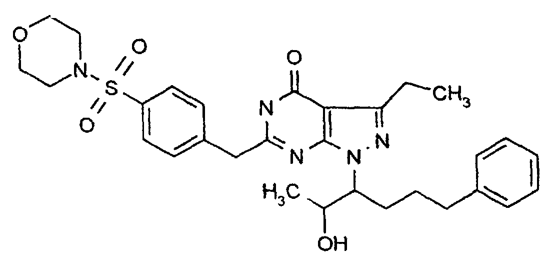

6 mg (0,02 mmol) 5-Amino-4-acetamido-3-ethyl-1-[2-hydroxy-6-phenyl-hex-3-yl]-pyrazol (Diastereomerengemisch) werden zusammen mit ca. 20 mg (0,08 mmol) 4-Morpholinosulfonylphenylessigester und 0,2 ml (0,1 mmol) 0,5 molarer NaOEt in EtOH 1,5 h unter Argon am Rückfluß erhitzt. Der Ansatz wird mit 0,5 ml Dichlormethan und 0,5 ml 10% Natriumhydrogencarbonatlösung versetzt und kräftig gerührt. Die organische Phase wird abgetrennt und durch Chromatographie an Kieselgel gereinigt. Man erhält so eine saubere Fraktion:
2,0 mg (= 18 % d.Th.) 6-(4-Morpholinosulfonylbenzyl)-3-ethyl-1-[2-hydroxy-(6-phenyl)-hex-3-yl]-pyrazolo[3,4-*d*]pyrimidin-4(5H)-on (Diastereomerengemisch)
(DC R_{f}-Wert = 0,61; Laufmittel: Dichlormethan/Methanol 10:1, Merck Si60 Art.-Nr. 1.05719).

## Patentansprüche

1. 1,5-Dihydro-pyrazolo[3,4-b]-pyrimidinon-derivate der allgemeinen Formel (I) 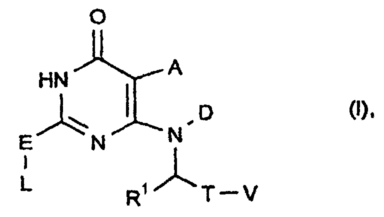 in welcher
A und D gemeinsam für einen Rest der Formel 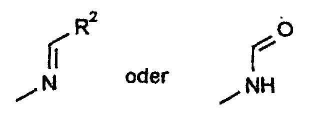 stehen
worin
R² Aryl bis 6 bis 10 Kohlenstoffatomen bedeutet, das gegebenenfalls bis zu 3-fach gleich oder verschieden durch Nitro, Cyano, Hydroxyl, Trifluormethyl, Halogen, Carboxyl oder durch geradkettiges oder verzweigtes Acyl, Alkoxy oder Alkoxycarbonyl mit jeweils bis zu 6 Kohlenstoffatomen substituiert ist, oder
Wasserstoff, Trifluormethyl, Cyano, Carboxyl, oder geradkettiges oder verzweigtes Alkoxy oder Alkoxycarbonyl mit jeweils bis zu 8 Kohlenstoffatomen oder geradkettiges oder verzweigtes Alkyl mit bis zu 8 Kohlenstoffatomen bedeutet, das gegebenenfalls durch Hydroxy substituiert ist,
R¹ für geradkettiges oder verzweigtes Acyl mit bis zu 4 Kohlenstoffatomen steht, oder
für geradkettiges oder verzweigtes Alkyl mit bis zu 10 Kohlenstoffatomen steht, das gegebenenfalls durch Hydroxy, Azido oder durch eine Gruppe der Formel -NR³R⁴ oder -OSO₂R⁵ substituiert ist,
worin
R³ und R⁴ gleich oder verschieden sind und
Wasserstoff oder geradkettiges oder verzweigtes Alkyl mit bis zu 6 Kohlenstoffatomen bedeuten, oder
R³ und R⁴ gemeinsam mit dem Stickstoffatom einen 5 oder 6-gliedrigen gesättigten Heterocyclus bilden, der gegebenenfalls ein weiteres Heteroatom aus der Reihe S oder 0 oder einen Rest -NR⁶ enthalten kann,
worin
R⁶ Wasserstoff oder geradkettiges oder verzweigtes Alkyl ist bis zu 4 Kohlenstoffatomen bedeuten,
und
R⁵ Phenyl oder geradkettiges oder verzweigtes Alkyl mit bis zu 5 Kohlenstoffatomen bedeutet,
E für eine geradkettige oder verzweigte Alkylen- oder Alkenylenkette mit jeweils bis zu 6 Kohlenstoffatomen steht, die gegebenenfalls durch Hydroxy substituiert sind, oder
für die C=O-Gruppe steht,
L und V gleich oder verschieden sind und für Aryl mit 6 bis 10 Kohlenstoffatomen. oder für einen 5- bis 7-gliedrigen aromatischen, gegebenenfalls benzokondensierten Heterocyclus mit bis zu 3 Heteroatomen aus der Reihe S, N und/oder O stehen, die gegebenenfalls bis zu 3-fach gleich oder verschieden durch Halogen, Hydröxy, Nitro, Trifluormethyl, Carboxy, geradkettiges oder verzweigtes Alkyl, Alkoxy oder Alkoxycarbonyl mit jeweils bis zu 6 Kohlenstoffatomen oder durch eine Gruppe der Formel -(W)ₐ-NR⁷R⁸ substituiert sind,
worin
a eine Zahl 0 oder 1 bedeutet,
W einen Rest der Formel -CO oder -SO₂ bedeutet,
R⁷ und R⁸ gleich oder verschieden sind und die oben angegebene Bedeutung von R³ und R⁴ haben,
und/oder die Cyclen gegebenenfalls durch Aryl mit 6 bis 10 Kohlenstoffatomen oder durch einen 5- bis 7-gliedrigen aromatischen, gegebenenfalls benzokondensierten Heterocyclus mit bis zu 3 Heteroatomen aus der Reihe S, N und/oder O substituiert sind, die ihrerseits gegebenenfalls bis zu 2-fach gleich oder verschieden durch Halogen, Hydroxy, Nitro, Carboxyl, Trifluormethyl oder durch geradkettiges oder verzweigtes Alkyl, Alkox oder Alkoxycarbonyl mit jeweils bis zu 5 Kohlenstoffatomen oder durch eine Gruppe der Formel -(W')_{b} -NR⁹R¹⁰ substituiert sind,
worin
b die oben angegebene Bedeutung von a hat und mit dieser gleich oder verschieden ist,
R⁹ und R¹⁰ die oben angegebene Bedeutung von R³ und R⁴ haben und mit dieser gleich oder verschieden sind,
W' die oben angegebene Bedeutung von W hat und mit dieser gleich oder verschieden ist,
oder
L für einen Rest der Formel 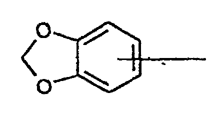 steht,
T für einen Rest der Formel -CH₂-X-Y- steht,
worin
X eine Bindung bedeutet oder ein Sauerstoff- oder Schwefelatom oder die -NH-Gruppe bedeutet,
Y eine geradkettige oder. verzweigte Alkylenkette mit bis zu 9 Kohlenstoffatomen bedeutet,
und deren Tautomere und Salze.

2. 1,5-Dihydro-pyrazolo[3,4-b]-pyrimidinon-derivate der Formel nach Anspruch 1,
in welcher
A und D gemeinsam für einen Rest der Formel 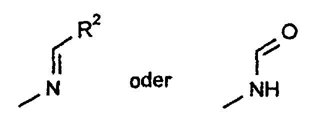 stehen
worin
R² Phenyl bedeutet, das gegebenenfalls bis zu 2-fach gleich oder verschieden durch Nitro, Cyano, Hxdroxyl, Trifluormethyl, Fluor, Chlor, Brom, Carboxyl oder durch geradkettiges oder verzweigtes Acyl, Alkoxy oder Alkoxycarbonyl mit jeweils bis zu 5 Kohlenstoffatomen substituiert ist, oder
Wasserstoff, Trifluormethyl, Cyano, Carboxyl, geradkettiges oder verzweigtes Alkoxy oder Alkoxycarbonyl mit jeweils bis zu 6 Kohlenstoffatomen oder geradkettiges oder verzweigtes Alkyl mit bis zu 6 Kohlenstoffatomen bedeutet, das gegebenenfalls durch Hydroxy substituiert ist,
R¹ für geradkettiges oder verzweigtes Acyl mit bis zu 6 Kohlenstoffatomen steht, oder
für geradkettiges oder verzweigtes Alkyl mit bis zu 8 Kohlenstoffatomen steht, das gegebenenfalls durch Hydroxy, Azido oder durch eine Gruppe der Formel -NR³R⁴ oder O-SO₂-R⁵ substituiert ist,
worin
R³ und R⁴ gleich oder verschieden sind und
Wasserstoff oder geradkettiges oder verzweigtes Alkyl mit bis zu 5 Kohlenstoffatomen bedeuten, oder
R³ und R⁴ gemeinsam mit dem Stickstoffatom einen Morpholinyl-Piperidinyl- oder Piperazinylring bilden, wobei letzterer gegebenenfalls über die Stickstofffunktion durch geradkettiges oder verzweigtes Alkyl mit bis zu 3 Kohlenstoffatomen substituiert ist,
und
R⁵ Phenyl oder geradkettiges oder verzweigtes Alkyl mit bis zu 4 Kohlenstoffatomen bedeutet,
E für eine geradkettige oder verzweigte Alkylen- oder Alkenylenkette mit jeweils bis zu 5 Kohlenstoffatomen steht, die gegebenenfalls durch Hydroxy substituiert sind, oder
für die C=O-Gruppe steht,
L und V gleich oder verschieden sind und fiir Phenyl, Naphthyl; Pyridyl, Thienyl, Indolyl oder Furyl stehen, die gegebenenfalls bis zu 3-fach gleich oder verschieden durch Fluor, Chlor, Brom, Trifluormethyl, Hydroxy, Nitro, Carboxy, geradkettiges oder verzweigtes Alkyl, Alkoxy oder Alkoxycarbonyl mit jeweils bis zu 5 Kohlenstoffatomen oder durch eine Gruppe der Formel -(W)ₐNR⁷R⁸ substituiert ist,
worin
a eine Zahl 0 oder 1 bedeutet,
W einen Rest der Formel -CO oder -SO₂ bedeutet,
R⁷ und R⁸ gleich oder verschieden sind und die oben angegebene Bedeutung von R³ and R⁴ haben,
und/oder die Cyclen gegebenenfalls durch Naphthyl, Phenyl, Pyridyl, Indolyl, Thienyl oder Furyl gegebenenfalls durch Phenyl, Naphthyl, Pyridyl, Thienyl, Furyl, Pyrryl oder Pyrimidyl substituiert sind, die ihrerseits gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(W')_{b}NR⁹R¹⁰ substituiert sind,
worin
b die oben angegebene Bedeutung von a hat und mit dieser gleich oder verschieden ist,
W' die oben angegebene Bedeutung von W hat und mit dieser gleich odes verschieden ist,
R⁹ and R¹⁰ die oben angegebene Bedeutung von R³ und R⁴ haben,
oder
L für einen Rest der Formel 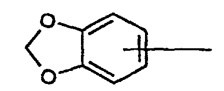 steht,
T für einen Rest der Formel -CH₂-X-Y- steht,
worin
X eine Bindung bedeutet oder ein Sauerstoff- oder Schwefelatom oder die -NH-Gruppe bedeutet,
Y eine geradkettige oder verzweigte Alkylenkette mit bis zu 8 Kohlenstoffatomen bedeutet,
und deren Tautomere und Salze.

3. 1,5-Dihydro-pyrazolo[3,4-b]-pyrimidinon-derivate der Formel (I) nach Anspruch 1
in welcher
A und D gemeinsam für einen Rest der Formel 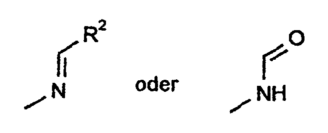 stehen
worin
R² Phenyl bedeutet, das gegebenenfalls bis zu 2-fach gleich oder verschieden durch Nitro, Cyano, Hxdroxyl, Trifluormethyl, Fluor, Chlor, Brom, Carboxyl oder durch geradkettiges oder verzweigtes Acyl, Akoxy oder Alkoxycarbonyl mit jeweils bis zu 4 Kohlenstoffatomen substituiert ist, oder
Wasserstoff, Trifluormethyl, Cyano, Carboxyl, geradkettiges oder verzweigtes Alkoxy oder Alkoxycarbonyl mit jeweils bis zu 5 Kohlenstoffatomen oder geradkettiges oder verzweigtes Alkyl mit bis zu 5 Kohlenstoffatomen bedeutet, das gegebenenfalls durch Hydroxy substituiert ist,
R¹ für geradkettiges oder verzweigtes Acyl mit bis zu 5 Kohlenstoffatomen steht, oder
für geradkettiges oder verzweigtes Alkyl mit bis zu 6 Kohlenstoffatomen steht, das gegebenenfalls durch Hydroxy, Azido oder durch eine Gruppe der Formel-NR³R⁴ oder O-SO₂R⁵ substituiert ist,
worin
R³ and R⁴ gleich oder verschieden sind und Wasserstoff oder geradkettiges oder verzweigtes Alkyl mit bis zu 4 Kohlenstoffatomen bedeuten, oder
R³ und R⁴ gemeinsam mit dem Stickstoffatom einen Morpholinyl-, Piperdinyl- oder Piperazinylring bilden, wobei letzterer gegebenenfalls über die Stickstofffunktion durch Methyl substituiert ist,
und
R⁵ Phenyl oder geradkettiges oder verzweigtes Alkyl mit bis zu 3 Kohlenstoffatomen bedeutet,
E für eine geradkettige oder verzweigte Alkylen- oder Alkenylenkette mit jeweils bis zu 5 Kohlenstoffatomen steht, die gegebenenfalls durch Hydroxy substituiert sind, oder
für die C=O-Gruppe steht,
L und V gleich oder verschieden sind und für Phenyl, Naphthyl, Furyl, Thienyl, Indolyl oder Pyridyl stehen, die gegebenenfalls bis zu 3-fach gleich oder verschieden durch Fluor, Chlor, Brom, Hydroxy, Nitro, Carboxy, geradkettiges oder verzweigtes Alkyl, Alkoxy oder Alkoxycarbonyl mit jeweils bis zu 4 Kohlenstoffatomen oder durch eine Gruppe der Formel -(W)ₐNR⁷R⁸ substituiert sind,
worin
a eine Zahl 0 oder 1 bedeutet,
W einen Rest der Formel -CO oder -SO₂ bedeutet,
R⁷ und R⁸ gleich oder verschieden sind und die oben angegebene Bedeutung von R³ und R⁴ haben,
und/oder die Cyclen gegebenenfalls durch Phenyl, Pyridyl, Thienyl oder Furyl substituiert sind, die ihrerseits gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(W')_{b}NR⁹R¹⁰ substituiert sind,
worin
b die oben angegebene Bedeutung von a hat und mit dieser gleich oder verschieden ist,
W' die oben angegebene Bedeutung von W hat und mit dieser gleich oder verschieden ist,
R⁹ und R¹⁰ die oben angegebene Bedeutung von R³ und R⁴ haben,
oder
L für einen Rest der Formel 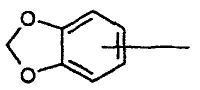 steht,
T für einen Rest der Formel -CH₂-X-Y- steht,
worin
X eine Bindung bedeutet oder ein Sauerstoff- oder Schwefelatom oder die -NH-Gruppe bedeutet,
Y eine geradkettige oder verzweigte Alkylenkette mit bis zu 6 Kohlenstoffatomen hedeutet
und deren Tautomere und Salze.

4. 1,5-Dihydro-pyrazolo[3,4-b]-pyrimidinon-derivate der Ansprüche 1 bis 3 als Arzneimittel.

5. Verfahren zur Herstellung von 1,5-Dihydro-pyrazolo[3,4-b]-pyrimidinon-derivate nach Anspruch 1 bis 3, **dadurch gekennzeichnet, daß** man
[A] im Fall A und D gemeinsam für den Rest der Formel 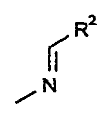 stehen,
zunächst Verbindungen der allgemeinen Formel (II) 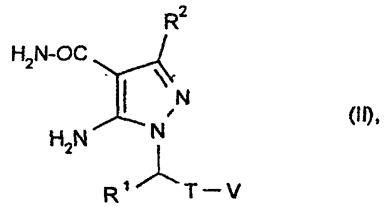 in welcher
R¹, R², T und VL die angegebene Bedeutung haben,
durch Umsetzung mit Verbindungen der allgemeinen Formel (III)
L-E-CO-Cl (III)
in welcher
E und L die angegebene Bedeutung haben,
in inerten Lösemitteln und in Anwesenheit einer Base,
in die Verbindungen der allgemeinen Formel (IV) 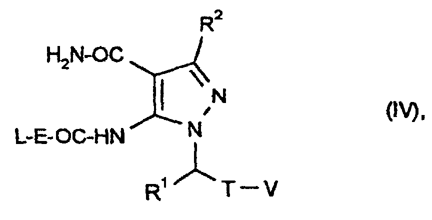 in welcher
E, L, T, V, R¹ und R² die angegebene Bedeutung haben,
überführt und anschließend mit Basen cyclisiert,
oder
[B] Verbindungen der allgemeinen Formel (II) unter direkter Cyclisierung mit Verbindungen der allgemeinen Formel (IIIa)
L-E-CO₂-R¹¹ (IIIa)
in welcher
E und L die oben angegebene Bedeutung haben,
und
R¹¹ für Methyl oder Ethyl steht,
umsetzt,
und in einem zweiten Schritt in inerten Lösemitteln und in Anwesenheit einer Base cyclisiert,
oder
[C] Verbindungen der allgemeinen Formel (V) 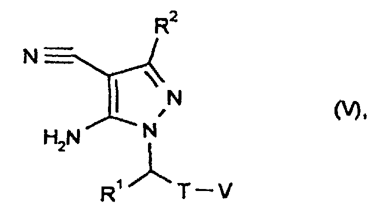 in welcher
R¹, R², T and V die oben angegebene Bedeutung haben,
zunächst durch Umsetzung mit Verbindungen der allgemeinen Formel (III) in inerten Lösemitteln und in Anwesenheit einer Base zu den Verbindungen der allgemeinen Formel (VI) 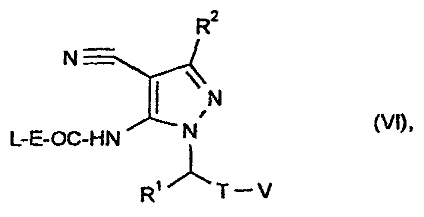 in welcher
R¹, R², E, L, T and V die oben angegebene Bedeutung haben,
umsetzt,
und in einem zweiten Schritt in inerten Lösemitteln und in Anwesenheit einer Base und eines Oxidationsmittels cyclisiert,
oder
[D] im Fall, daß A and D gemeinsam für den Rest der Formel 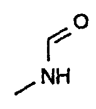 stehen,
die entsprechenden Verbindungen der allgemeinen Formel (I), in welcher R² für Methoxy steht, in dem System Natriumjodid / Trimethylchlorsilan in inerten Lösemitteln umsetzt,
und gegebenenfalls die unter R¹ aufgeführten Substituenten durch Folgereaktionen wie Acylierung, Oxidation, Substitution und/oder Reduktionen einführt bzw. derivatisiert,
und ebenso die oben unter L und V aufgeführten Substituenten nach üblichen Methoden einführt und/oder variiert.

6. Arzneimittel enthaltend mindestens ein 1,5-Dihydro-pyrazolo[3,4-b]-pyrimidinon-derivat nach Anspruch 1 bis 3 sowie pharmakologisch unbedenkliche Formulierungshilfsstoffe.

7. Arzneimittel nach Anspruch 6 zur Behandlung kardiovaskulärer und cerebrovaskulärer Erkrankungen, peripherer Gefäßerkrankungen sowie Erkrankungen des Urogenitaltraktes.

8. Arzneimittel nach Anspruch 6 und 7 zur Behandlung von Impotenz.

9. Verwendung von 1,5-Dihydro-pyrazolo[3,4-b]-pyrimidinon-derivaten nach Anspruch 1 bis 3 zur Herstellung von Arzneimitteln.

10. Verwendung nach Anspruch 9 zur Herstellung von Arzneimitteln zur Behandlung von kardiovakulären und cerebrovaskulären Erkrankungen, peripheren Gefäßerkrankungen sowie Erkrankungen des Urogenitaltraktes.

## Claims

1. 1,5-Dihydro-pyrazolo[3,4-d]-pyrimidinone derivatives of the general formula (I) 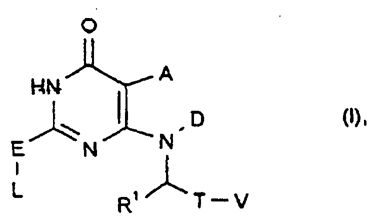 in which
A and D together represent a radical of the formula 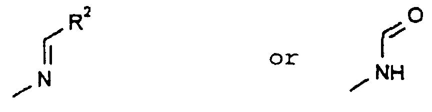 where
R² represents aryl which has 6 to 10 carbon atoms and which is optionally up to trisubstituted by identical or different substituents from the series consisting of nitro, cyano, hydroxyl, trifluoromethyl, halogen, carboxyl, or by straight-chain or branched acyl, alkoxy or alkoxycarbonyl, each of which has up to 6 carbon atoms or
hydrogen, trifluoromethyl, cyano, carboxyl, or straight-chain or branched alkoxy or alkoxycarbonyl, each of which has up to 8 carbon atoms, or straight-chain or branched alkyl which has up to 8 carbon atoms and which is optionally substituted by hydroxyl,
R¹ represents straight-chain or branched acyl having up to 4 carbon atoms,
or
represents straight-chain or branched alkyl having up to 10 carbon atoms, optionally substituted by hydroxyl, azido or by a group of the formula -NR³R⁴ or -OSO₂R⁵
where
R³ and R⁴ are identical or different and represent hydrogen or straight-chain or branched alkyl having up to 6 carbon atoms, or
R³ and R⁴ together with the nitrogen atom form a 5 or 6-membered saturated heterocycle which can optionally contain a further hetero atom selected from the series consisting of S or 0 or a radical -NR⁶
where
R⁶ represent hydrogen or straight-chain or branched alkyl is up to 4 carbon atoms
and
R⁵ represents phenyl or straight-chain or branched alkyl having up to 5 carbon atoms,
E represents a straight-chain or branched alkylene or alkenylene chain, each of which has up to 6 carbon atoms which are optionally substituted by hydroxyl, or represents the C=O group,
L and V are identical or different and represent aryl having 6 to 10 carbon atoms or a 5- to 7-membered aromatic, optionally benzo-fused, heterocycle which has up to 3 hetero atoms from the series consisting of S, N and/or O which are optionally up to trisubstituted by identical or different substituents from the series consisting of halogen, hydroxyl, nitro, trifluoromethyl, carboxyl, straight-chain or branched alkyl, alkoxy or alkoxycarbonyl, each of which has up to 6 carbon atoms, or by a group of the formula - (W)ₐ-NR⁷R⁸
where
a is a number 0 or 1,
W is a radical of the formula -CO or -SO₂,
R⁷ and R⁸ are identical or different and have the abovementioned meaning of R³ and R⁴,
and/or the cycles are optionally substituted by aryl having 6 to 10 carbon atoms or by a 5- to 7-membered aromatic, optionally benzo-fused, heterocycle having up to 3 hetero atoms from the series consisting of S, N and/or O, which, in turn, are optionally up to disubstituted by identical or different substituents from the series consisting of halogen, hydroxyl, nitro, carboxyl, trifluoromethyl or by straight-chain or branched alkyl, alkox or alkoxycarbonyl, each of which has up to 5 carbon atoms, or by a group of the formula -(W')_{b}-NR⁹R¹⁰
where
b has the abovementioned meaning of a and is identical to this meaning or different from it,
R⁹ and R¹⁰ have the abovementioned meaning of R³ and R⁴ and are identical to this meaning or different from it,
W' has the abovementioned meaning of W and is identical to this meaning or different from it,
or
L represents a radical of the formula 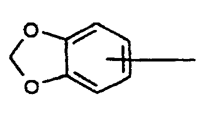
T represents a radical of the formula -CH₂-X-Y-
where
X represents a bond or an oxygen or sulphur atom or the -NH-group,
Y represents a straight-chain or branched alkylene chain having up to 9 carbon atoms,
and the tautomers and salts of these.

2. 1, 5-Dihydro-pyrazolo[3,4-d]-pyrimidinone derivatives of the formula according to Claim 1
in which
A and D together represent a radical of the formula 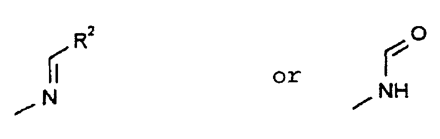 where
R² represents phenyl which is optionally up to disubstituted by identical or different substituents from the series consisting of nitro, cyano, hxdroxyl, trifluoromethyl, fluorine, chlorine, bromine, carboxyl or by straight-chain or branched acyl, alkoxy or alkoxycarbonyl, each of which has up to 5 carbon atoms, or
hydrogen, trifluoromethyl, cyano, carboxyl, straight-chain or branched alkoxy or alkoxycarbonyl, each of which has up to 6 carbon atoms, or straight-chain or branched alkyl which has up to 6 carbon atoms and which is optionally substituted by hydroxyl,
R¹ represents straight-chain or branched acyl having up to 6 carbon atoms, or
straight-chain or branched alkyl which has up to 8 carbon atoms and which is optionally substituted by hydroxyl, azido or by a group of the formula -NR³R⁴ or O-SO₂-R⁵
in which
R³ and R⁴ are identical or different and represent
hydrogen or straight-chain or branched alkyl having up to 5 carbon atoms, or
R³ and R⁴ together with the nitrogen atom form a morpholinyl-piperidinyl or piperazinyl ring, the latter optionally being substituted via the nitrogen function by straight-chain or branched alkyl having up to 3 carbon atoms,
and
R⁵ represents phenyl or straight-chain or branched alkyl having up to 4 carbon atoms,
E represents a straight-chain or branched alkylene or alkenylene chain, each of which has up to 5 carbon atoms and each of which is optionally substituted by hydroxyl, or represents the C=O group,
L and V are identical or different and represent phenyl, naphthyl, pyridyl, thienyl, indolyl or furyl, each of which is up to trisubstituted by identical or different substituents from the series consisting of fluorine, chlorine, bromine, trifluoromethyl, hydroxyl, nitro, carboxyl, straight-chain or branched alkyl, alkoxy or alkoxycarbonyl, each of which has up to 5 carbon atoms, or by a group of the formula -(W)ₐNR⁷R⁸
in which
a represents a number 0 or 1,
W represents a radical of the formula -CO or -SO₂,
R⁷ and R⁸ are identical or different and have the abovementioned meaning of R³ and R⁴,
and/or the cycles are optionally substituted by naphthyl, phenyl, pyridyl, indolyl, thienyl or furyl, optionally by phenyl, naphthyl, pyridyl, thienyl, furyl, pyrryl or pyrimidyl, which, in turn, are optionally substituted by fluorine, chlorine, bromine, hydroxyl, nitro, carboxyl, trifluoromethyl or straight-chain or branched alkyl, alkoxy or alkoxycarbonyl, each of which has up to 3 carbon atoms, or by a group of the formula -(W')_{b}NR⁹R¹⁰
in which
b has the abovementioned meaning of a and is identical to this meaning or different from it,
W' has the abovementioned meaning of W and is identical to this meaning or different from it,
R⁹ and R¹⁰ have the abovementioned meaning of R³ and R⁴,
or
L represents a radical of the formula 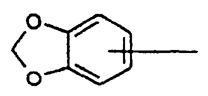
T represents a radical of the formula -CH₂-X-Y-
in which
X represents a bond or an oxygen or sulphur atom or the -NH- group,
Y represents a straight-chain or branched alkylene chain having up to 8 carbon atoms,
and the tautomers and salts of these.

3. 1,5-Dihydro-pyrazolo[3,4-d]-pyrimidinone derivatives of the formula (I) according to Claim 1
in which
A and D together represent a radical of the formula 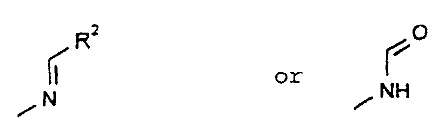 in which
R² represents phenyl which is optionally up to disubstituted by identical or different substituents from the series consisting of vitro, cyano, hxdroxyl, trifluoromethyl, fluorine, chlorine, bromine, carboxyl or by straight-chain or branched acyl, akoxy or alkoxycarbonyl, each of which has up to 4 carbon atoms,
or
represents hydrogen, trifluoromethyl, cyano, carboxyl, straight-chain or branched alkoxy or alkoxycarbonyl, each of which has up to 5 carbon atoms, or straight-chain or branched alkyl which has up to 5 carbon atoms and which is optionally substituted by hydroxyl,
R¹ represents straight-chain or branched acyl having up to 5 carbon atoms, or
straight-chain or branched alkyl which has up to 6 carbon atoms and which is optionally substituted by hydroxyl, azido or by a group of the formula -NR³R⁴ or O-SO₂R⁵
in which
R³ and R⁴ are identical or different and represent hydrogen or straight-chain or branched alkyl having up to 4 carbon atoms, or
R³ and R⁴ together with the nitrogen atom form a morpholinyl, piperdinyl or piperazinyl ring, the latter optionally being methyl-substituted via the nitrogen function,
and
R⁵ represents phenyl or straight-chain or branched alkyl having up to 3 carbon atoms,
E represents a straight-chain or branched alkylene or alkenylene chain, each of which has up to 5 carbon atoms which are optionally substituted by hydroxyl, or represents the C=O group,
L and V are identical or different and represent phenyl, naphthyl, furyl, thienyl, indolyl or pyridyl, each of which is optionally up to trisubstituted by identical or different substituents from the series consisting of fluorine, chlorine, bromine, hydroxyl, nitro, carboxyl, straight-chain or branched alkyl, alkoxy or alkoxycarbonyl, each of which has up to 4 carbon atoms, or by a group of the formula -(W)ₐNR⁷R⁸
in which
a represents a number 0 or 1,
W represents a radical of the formula -CO or-SO₂,
R⁷ and R⁸ are identical or different and have the abovementioned meaning of R³ and R⁴,
and/or the cycles are optionally substituted by phenyl, pyridyl, thienyl or furyl, which, in turn, are optionally substituted by fluorine, chlorine, bromine, hydroxyl, nitro, carboxyl, trifluoromethyl or by straight-chain or branched alkyl, alkoxy or alkoxycarbonyl, each of which has up to 3 carbon atoms, or by a group of the formula -(W')_{b}NR⁹R¹⁰
in which
b has the abovementioned meaning of a and is identical to this meaning or different from it,
W' has the abovementioned meaning of W and is identical to this meaning or different from it,
R⁹ and R¹⁰ have the abovementioned meaning of R³ and R⁴,
or
L represents a radical of the formula 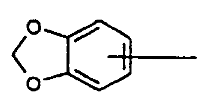
T represents a radical of the formula -CH₂-X-Y-
in which
X represents a bond or an oxygen or sulphur atom or the -NH-group,
Y represents a straight-chain or branched alkylene chain having up to 6 carbon atoms,
and the tautomers and salts of these.

4. 1,5-Dihydro-pyrazolo[3,4-d]-pyrimidinone derivatives of Claims 1 to 3 as medicaments.

5. Process for the preparation of 1,5-dihydropyrazolo[3,4-d]-pyrimidinone derivatives according to Claim 1 to 3, **characterized in that**
[A] in the event that A and D together represent the radical of the formula 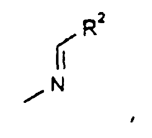 compounds of the general formula (II) 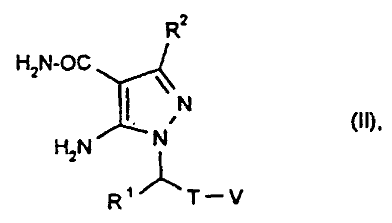 in which
R¹, R², T and V have the abovementioned meaning
are firstly converted into the compounds of the general formula (IV) 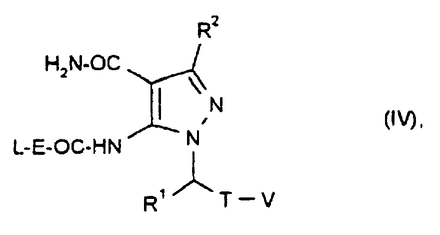 in which
E, L, T, V, R¹ and R² have the abovementioned meaning
by reacting them, in inert solvents and in the presence of a base, with compounds of the general formula (III)
L-E-CO-Cl (III)
in which
E and L have the abovementioned meaning
and subsequently cyclizing the product with bases,
or
[B] compounds of the general formula (II) are reacted, with direct cyclization, with compounds of the general formula (IIIa)
L-E-CO₂-R¹¹ (IIIa)
in which
E and L have the abovementioned meaning
and
R¹¹ represents methyl or ethyl,
and, in a second step, the product is cyclized in inert solvents and in the presence of a base,
or
[C] compounds of the general formula (V) 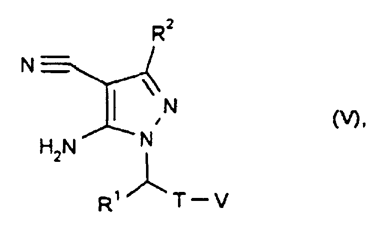 in which
R¹, R², T and V have the abovementioned meaning
are first reacted with compounds of the general formula (III) in inert solvent and in the presence of a base to give the compounds of the general formula (VI) 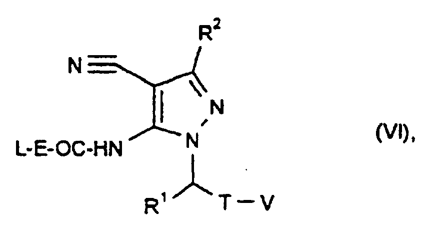 in which
R¹, R², E, L, T and V have the abovementioned meaning,
and, in a 2nd step, cyclizing the product in inert solvents and in the presence of a base and of an oxidant,
or
[D] in the event that A and D together represent the radical of the formula 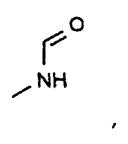 the corresponding compounds of the general formula (I) in which R² represents methoxy is reacted in the system sodium iodide/trimethylchlorosilane in inert solvents,
and, if appropriate, the substituents mentioned under R¹ are introduced or derivatized by subsequent reactions such as acylation, oxidation, substitution and/or reductions,
and, equally, the substituents mentioned above under L and V are introduced and/or varied by customary methods.

6. Medicament containing at least one 1,5-dihydropyrazolo[3,4-d]-pyrimidinone derivative according to Claim 1 to 3 and pharmacologically acceptable formulation auxiliaries.

7. Medicament according to Claim 6 for the treatment of cardiovascular and cerebrovascular diseases, diseases of the peripheral blood vessels and diseases of the urogenital tract.

8. Medicament according to Claim 6 and 7 for the treatment of impotence.

9. Use of 1,5-dihydro-pyrazolo[3,4-d]-pyrimidinone derivatives according to Claim 1 to 3 for the preparation of medicaments.

10. Use according to Claim 9 for the preparation of medicaments for the treatment of cardiovascular and cerebrovascular diseases, diseases of the peripheral blood vessels and diseases of the urogenital tract.

## Revendications

1. Dérivés de 1,5-dihydropyrazolo[3, 4-b]pyrimidinone de la formule générale (I) : 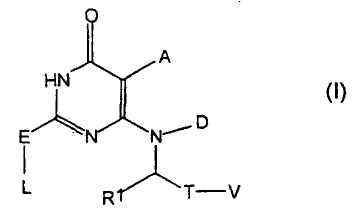 dans laquelle :
A et D représentent ensemble, un reste de la formule : 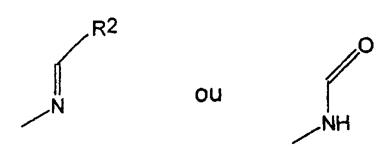 où
R² représente un radical aryle ayant 6 à 10 atomes de carbone, qui est le cas échéant substitué jusqu'à 3 fois, de manière identique ou différente, par le radical nitro, cyano, hydroxyle, trifluorométhyle, un atome d'halogène, le radical carboxyle ou par un radical acyle, alcoxy ou alcoxycarbonyle linéaire ou ramifié, ayant chaque fois jusqu'à 6 atomes de carbone, ou l'atome d'hydrogène, le radical trifluorométhyle, cyano, carboxyle ou un radical alcoxy ou alcoxycarbonyle linéaire ou ramifié, ayant chaque fois jusqu'à 8 atomes de carbone ou un radical alcoyle linéaire ou ramifié, ayant jusqu'à 8 atomes de carbone, qui est le cas échéant, substitué par le radical hydroxyle ;
R¹ représente un radical acyle, linéaire ou ramifié, ayant jusqu'à 4 atomes de carbone, ou un radical alcoyle linéaire ou ramifié, ayant jusqu'à 10 atomes de carbone, qui est le cas échéant, substitué par le radical hydroxyle; azido ou par un groupe de la formule -NR³R⁴ ou -OSO₂R⁵ ;
où
R³ et R⁴ sont identiques ou différents et représentent l'atome d'hydrogène ou un radical alcoyle linéaire ou ramifié, ayant jusqu'à 6 atomes de carbone, ou
R³ et R⁴ forment ensemble, avec l'atome d'azote, un hétérocyle saturé à 5 ou 6 membres, qui peut contenir le cas échéant, un autre hétéroatome de la série S ou O ou un reste -NR⁶, où
R⁶ représente l'atome d'hydrogène ou un radical alcoyle linéaire ou ramifié, ayant jusqu'à 4 atomes de carbone, et
R⁵ représente le radical phényle ou un radical alcoyle linéaire ou ramifié, ayant jusqu'à 5 atomes de carbone ;
E représente une chaine alcoylène ou alcénylène linéaire ou ramifiée, ayant chaque fois jusqu'à 6 atomes de carbone, qui est le cas échéant, substituée par le radical hydroxy, ou le groupe C=O ;
L et V sont identiques ou différents et représentent un radical aryle ayant 6 à 10 atomes de carbone ou un hétérocycle aromatique ayant 5 à 7 membres, le cas échéant condensé au benzène, ayant jusqu'à 3 hétéroatomes de la série S, N et/ou O, qui est le cas échéant, substitué jusqu'à 3 fois, de manière identique ou différente par un atome d'halogène, le radical hydroxyle, nitro, trifluorométhyle, carboxyle, un radical alcoyle, alcoxy ou alcoxycarbonyle linéaire ou ramifié, ayant jusqu'à 6 atomes de carbone, ou par un groupe de la formule - (W)ₐ-NR⁷R⁸ ;
où
a représente le nombre 0 ou 1 ;
W représente un reste de la formule -CO ou -SO₂ ;
R⁷ et R⁸ sont identiques ou différents et ont la signification indiquée ci-dessus pour R³ et R⁴ ;
et/ou les cycles sont le cas échéant substitués par un radical aryle ayant 6 à 10 atomes de carbone ou par un hétérocycle aromatique ayant 5 à 7 membres, le cas échéant condensé au benzène, ayant jusqu'à 3 hétéroatomes de la série S, N et/ou O, qui est pour sa part, le cas échéant, substitué jusqu'à 2 fois, de manière identique ou différente par un atome d'halogène, le radical hydroxyle, nitro, carboxyle, trifluorométhyle ou par un radical alcoyle, alcoxy ou alcoxycarbonyle linéaire ou ramifié, ayant jusqu'à 5 atomes de carbone, ou par un groupe de la formule -(W')_{b}-NR⁹R¹⁰ ;
où
b a la signification indiquée ci-dessus pour a et est identique à ou différent de celui-ci ;
R⁹ et R¹⁰ ont la signification indiquée ci-dessus pour R³ et R⁴ et sont identiques à ou différents de ceux-ci ;
W' a la signification indiquée ci-dessus pour W et est identique à ou différent de celui-ci ;
ou
L représente un reste de la formule : 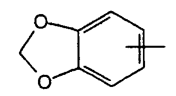
T représente un reste de la formule -CH₂-X-Y,
où
X représente une liaison ou l'atome d'oxygène ou de soufre ou le radical -NH ;
Y représente une chaine alcoylène linéaire ou ramifiée ayant jusqu'à 9 atomes de carbone,
et leurs tautomères et sels.

2. Dérivés de 1, 5-dihydropyrazolo[3, 4-b]pyrimidinone de la formule (I) suivant la revendication 1,
dans laquelle :
A et D représentent ensemble, un reste de la formule : 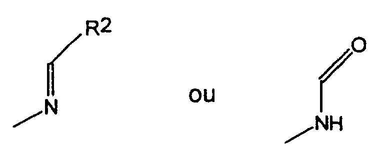 où
R² représente le radical phényle, qui est le cas échéant substitué jusqu'à 2 fois, de manière identique ou différente, par le radical nitro, cyano, hydroxyle, trifluorométhyle, l'atome de fluor, de chlore, de brome, le radical carboxyle ou par un radical acyle, alcoxy ou alcoxycarbonyle linéaire ou ramifié, ayant chaque fois jusqu'à 5 atomes de carbone, ou l'atome d'hydrogène, le radical trifluorométhyle, cyano, carboxyle ou un radical alcoxy ou alcoxycarbonyle linéaire ou ramifié, ayant chaque fois jusqu'à 6 atomes de carbone ou un radical alcoyle linéaire ou ramifié, ayant jusqu'à 6 atomes de carbone, qui est le cas échéant, substitué par le radical hydroxyle ;
R¹ représente un radical acyle, linéaire ou ramifié, ayant jusqu'à 6 atomes de carbone, ou un radical alcoyle linéaire ou ramifié, ayant jusqu'à 8 atomes de carbone, qui est le cas échéant, substitué par le radical hydroxyle, azido ou par un groupe de la formule -NR³R⁴ ou -OSO₂R⁵ ;
où
R³ et R⁴ sont identiques ou différents et représentent l'atome d'hydrogène ou un radical alcoyle linéaire ou ramifié, ayant jusqu'à 5 atomes de carbone, ou
R³ et R⁴ forment ensemble; avec l'atome d'azote, le radical morpholinyle, pipéridinyle ou pipérazinyle, où ce dernier est le cas échéant substitué sur la fonction azote, par un radical alcoyle linéaire ou ramifié ayant jusqu'à 3 atomes de carbone, et
R⁵ représente le radical phényle ou un radical alcoyle linéaire ou ramifié, ayant jusqu'à 4 atomes de carbone ;
E représente une chaine alcoylène ou alcénylène linéaire ou ramifiée, ayant chaque fois jusqu'à 5 atomes de carbone, qui est le cas échéant, substituée par le radical hydroxyle, ou le groupe C=O ;
L et V sont identiques ou différents et représentent le radical phényle, naphtyle, pyridyle, thiényle, indolyle ou furyle, qui est le cas échéant, substitué jusqu'à 3 fois, de manière identique ou différente par l'atome de fluor, de chlore, de brome, le radical trifluorométhyle, hydroxyle, nitro, carboxyle, un radical alcoyle, alcoxy ou alcoxycarbonyle linéaire ou ramifié, ayant jusqu'à 5 atomes de carbone, ou par un groupe de la formule - (W)ₐ-NR⁷R⁸ ;
où
a représente le nombre 0 ou 1 ;
W représente un reste de la formule -CO ou -SO₂ ;
R⁷ et R⁸ sont identiques ou différents et ont la signification indiquée ci-dessus pour R³ et R⁴ ;
et/ou les cycles sont le cas échéant substitués par les radicaux naphtyle, phényle, pyridyle, indolyle, thiényle, furyle, pyrryle ou pyrimidyle, qui sont substitués pour leur part, le cas échéant, par l'atome de fluor, de chlore, de brome, le radical hydroxyle, nitro, carboxyle, trifluorométhyle ou par un radical alcoyle, alcoxy ou alcoxycarbonyle linéaire ou ramifié, ayant jusqu'à 3 atomes de carbone, ou par un groupe de la formule -(W')_{b}-NR⁹R¹⁰ ;
où
b a la signification indiquée ci-dessus pour a et est identique à ou différent de celui-ci ;
W' a la signification indiquée ci-dessus pour W et est identique à ou différent de celui-ci ;
R⁹ et R¹⁰ ont la signification indiquée ci-dessus pour R³ et R⁴ ;
ou
L représente un reste de la formule : 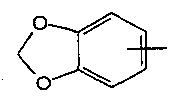
T représente un reste de la formule -CH₂-X-Y,
où
X représente une liaison ou l'atome d'oxygène ou de soufre ou le radical -NH ;
Y représente une chaine alcoylène linéaire ou ramifiée ayant jusqu'à 8 atomes de carbone,
et leurs tautomères et sels.

3. Dérivés de 1,5-dihydropyrazolo[3,4-b]pyrimidinone de la formule (I) suivant la revendication 1,
dans laquelle :
A et D représentent ensemble, un reste de la formule : 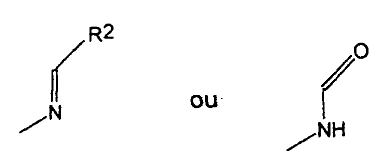 où
R² représente le radical phényle, qui est le cas échéant substitué jusqu'à 2 fois, de manière identique ou différente, par le radical nitro, cyano, hydroxyle, trifluorométhyle, l'atome de fluor, de chlore, de brome, le radical carboxyle ou par un radical acyle, alcoxy ou alcoxycarbonyle linéaire ou ramifié, ayant chaque fois jusqu'à 4 atomes de carbone, ou l'atome d'hydrogène, le radical trifluorométhyle, cyano, carboxyle ou un radical alcoxy ou alcoxycarbonyle linéaire ou ramifié, ayant chaque fois jusqu'à 5 atomes de carbone ou un radical alcoyle linéaire ou ramifié, ayant jusqu'à 5 atomes de carbone, qui est le cas échéant, substitué par le radical hydroxyle ;
R¹ représente un radical acyle, linéaire ou ramifié, ayant jusqu'à 5 atomes de carbone, ou un radical alcoyle linéaire ou ramifié, ayant jusqu'à 6 atomes de carbone, qui est le cas échéant, substitué par le radical hydroxyle, azido ou par un groupe de la formule -NR³R⁴ ou -OSO₂R⁵ ;
où
R³ et R⁴ sont identiques ou différents et représentent l'atome d'hydrogène ou un radical alcoyle linéaire ou ramifié, ayant jusqu'à 4 atomes de carbone, ou
R³ et R⁴ forment ensemble, avec l'atome d'azote, le cycle morpholinyle, pipéridinyle ou pipérazinyle, où ce dernier est le cas échéant substitué sur la fonction azote, par le radical méthyle, et
R⁵ représente le radical phényle ou un radical alcoyle linéaire ou ramifié, ayant jusqu'à 3 atomes de carbone ;
E représente une chaine alcoylène ou alcénylène linéaire ou ramifiée, ayant chaque fois jusqu'à 5 atomes de carbone, qui est le cas échéant, substituée par le radical hydroxyle, ou le groupe C=O ;
L et V sont identiques ou différents et représentent les radicaux phényle, naphtyle, furyle, thiényle, indolyle ou pyridyle; qui sont le cas échéant, substitués jusqu'à 3 fois, de manière identique ou différente par l'atome de fluor, de chlore, de brome, le radical hydroxyle, nitro, carboxyle, un radical alcoyle, alcoxy ou alcoxycarbonyle linéaire ou ramifié, ayant jusqu'à 4 atomes de carbone, ou par un groupe de la formule -(W)ₐ-NR⁷R⁸ ;
où
a représente le nombre, 0 ou 1 ;
W représente un reste de la formule -CO ou -SO₂ ;
R⁷ et R⁸ sont identiques ou différents et ont la signification indiquée ci-dessus pour R³ et R⁴ ;
et/ou les cycles sont le cas échéant substitués par les radicaux phényle, pyridyle, thiényle ou furyle, qui sont substitués pour leur part, le cas échéant, par l'atome de fluor, de chlore, de brome, le radical hydroxyle, nitro, carboxyle, trifluorométhyle ou par un radical alcoyle, alcoxy ou alcoxycarbonyle linéaire ou ramifié, ayant jusqu'à 3 atomes de carbone, ou par un groupe de la formule -(W')_{b}-NR⁹R¹⁰ ;
où
b a la signification indiquée ci-dessus pour a et est identique à ou différent de celui-ci ;
W' a la signification indiquée ci-dessus pour W et est identique à ou différent de celui-ci ;
R⁹ et R¹⁰ ont la signification indiquée ci-dessus pour R³ et R⁴ ;
ou
L représente un reste de la formule : 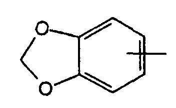
T représente un reste de la formule -CH₂-X-Y,
où
X représente une liaison ou l'atome d'oxygène ou de soufre ou le radical -NH ;
Y représente une chaine alcoylène linéaire ou ramifiée ayant jusqu' à 6 atomes de carbone,
et leurs tautomères et sels.

4. Dérivés de 1,5-dihydropyrazolo[3, 4-b]pyrimidinone suivant les revendications 1 à 3, comme agent pharmaceutique.

5. Procédé de préparation des dérivés de 1,5-dihydropyrazolo[3,4-b]pyrimidinone suivant les revendications 1 à 3, **caractérisé en ce que** :
[A] dans la cas où A et D représentent ensemble le reste de la formule : 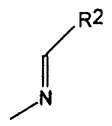 on convertit d'abord des composés de la formule générale (II) : 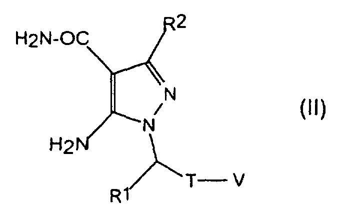 dans laquelle R¹, R², T et V ont la signification indiquée,
par réaction des composés de la formule générale (III) :
L - E - CO - Cl (III)
dans laquelle:
E et L ont la signification indiquée,
dans un solvant inerte et en présence d'une base,
en les composés de la formule générale (IV) : 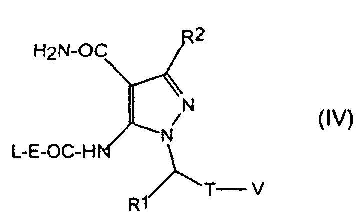 dans laquelle E, L, T, V, R¹ et R² ont la signification indiquée,
et ensuite, on cyclise avec une base, ou
[B] on fait réagir les composés de la formule générale (II) par cyclisation directe avec des composés de la formule générale (IIIa) :
L - E - CO₂ - R¹¹ (IIIa)
dans laquelle:
E et L ont la signification indiquée ci-dessus, et
R¹¹ représente le radical méthyle ou éthyle,
et dans une deuxième étape, on cyclise dans un solvant inerte en présence d'une base, ou
[C] on fait réagir les composés de la formule générale (V) : 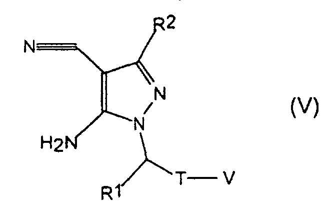 dans laquelle :
R¹, R², T et V ont la signification indiquée ci-dessus,
d'abord par réaction avec des composés de la formule générale (III) dans un solvant inerte et en présence d'une base en les composés de la formule générale (VI) : 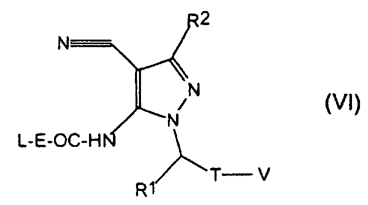 dans laquelle :
R¹, R², E, L, T et V ont la signification indiquée ci-dessus,
et dans une deuxième étape, on cyclise dans un solvant inerte et en présence d'une base et d'un agent d'oxydation, ou
[D] dans la cas où A et D représentent ensemble le reste de la formule : 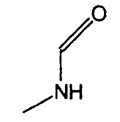 les composés correspondants de la formule générale (I), dans lesquels R² représente le radical méthoxy, sont mis à réagir dans le système iodure de sodium/triméthylchlorosilane dans un solvant inerte, et le cas échéant, les substituants indiqués sous R¹ sont convertis ou dérivatisés par des réactions ultérieures comme acylation, oxydation, substitution et/ou réduction,
et de même, les substituants indiqués sous L et V sont convertis et/ou variés par les procédés usuels.

6. Agent pharmaceutique contenant au moins un dérivé de 1,5-dihydropyrazolo[3,4-b]pyrimidinone suivant les revendications 1 à 3, ainsi que des auxiliaires de formulation pharmacologiquement sans inconvénient.

7. Agent pharmaceutique suivant la revendication 6, pour le traitement de maladies cardiovasculaires et cérébrovasculaires, des maladies des vaisseaux périphériques et des maladies de l'appareil urogénital.

8. Agent pharmaceutique suivant les revendications 6 et 7, pour le traitement de l'impuissance.

9. Utilisation de dérivés de 1,5-dihydropyrazolo[3,4-b]pyrimidinone suivant les revendications 1 à 3, pour la préparation d'agents pharmaceutiques.

10. Utilisation suivant la revendication 9, pour la préparation d'agents pharmaceutiques pour le traitement de maladies cardiovasculaires et cérébrovasculaires, des maladies des vaisseaux périphériques et des maladies de l'appareil urogénital.
